# EUROPEAN PATENT APPLICATION

(11) **EP 2 559 762 A1**
(43) Date of publication of application: **20.02.2013**
(21) Application number: 11768900.0
(22) Date of filing: 13.04.2011
(51) Int. Cl.: C12N 15/09, A61K 31/7115, A61K 39/00, A61K 39/39, A61K 39/395, A61P 35/00, C07K 16/30, C12N 5/10, C12P 21/08

(54) **NOVEL ANTI-HSP90 MONOCLONAL ANTIBODY**

(30) Priority: 13.04.2010 US 323578 P
(71) Applicant: Riken, Wako-shi, Saitama 351-0198 (JP)
(72) Inventor: UDONO, Heiichiro, Okayama-shi Okayama 700-8558 (JP); MIZUKAMI, Shusaku, Okayama-shi Okayama 700-8558 (JP)
(74) Representative: Duckworth, Timothy John
(86) International application number: PCT/JP2011/059213
(87) International publication number: WO 2011/129379

(57) **Abstract**

The present invention relates an anti-HSP90 antibody and use thereof, more specifically to an anti-HSP90 antibody capable of recognizing cell surface HSP90, a vaccine that utilizes the antibody, a drug that utilizes the antibody, an adjuvant comprising the antibody, a method of detecting a cell expressing HSP90 on the cell surface using the antibody, and the like.

## Description

### Technical Field

The present invention relates to an anti-HSP90 monoclonal antibody and use thereof, more specifically to an anti-HSP90 monoclonal antibody capable of recognizing cell surface HSP90, a method of detecting a cell expressing HSP90 on the cell surface using the antibody, a vaccine that utilizes the antibody, a drug that utilizes the antibody, an adjuvant comprising the antibody, and the like.

### Background Art

Heat shock proteins (HSPs) are major chaperone molecules whose expression levels are elevated due to a wide variety of stresses such as heat, and play pivotal roles in folding of newly synthesized proteins, prevention of damaged proteins from aggregation, promotion of proteasome-dependent degradation of irreversibly damaged proteins, and interorganelle and/or interprotein transportation of client proteins and peptides. Hsp90 is one of the most abundant chaperone proteins accounting for nearly 1% of total proteins in cells, and exists mainly in cytoplasm (non-patent documents 1 and 2).

However, it has recently been found in cancer cells that HSP90 exists on the surface of the cells and is involved as a mediator in invasion or metastasis of the cancer cells (non-patent documents 3 and 4). In addition, HSP90 is also expressed on the surface of nerve cells and is involved in cell migration in the developmental process (non-patent document 5). HSP90α is secreted from TGFα-stimulated human keratinocytes and interacts with LRP1, and thereby are involved in the motility of these cells (non-patent documents 6 and 7). These results suggest an additional role of HSP90 in which it is involved in a novel pathway that leads to cell migration and invasion when HSP90 exists on the cell surface.

Meanwhile, since there has been no appropriate monoclonal antibody capable of detecting cell surface HSP90 on dendritic cells (DCs) and macrophages, which are antigen-presenting cells (APCs), it has been difficult to identify HSP90 on the cell surface. Although all the preexisting anti-HSP90 antibodies well recognize HSP90 in cells, their reactivity with HSP90 on the cell membrane is low. For this reason, it has been impossible to date to detect a cell expressing HSP90 on the cell surface by any one of a variety of analytical methods such as flow cytometry.

It is known that, in dendritic cells, cross-presentation occurs, that is, antigens coming from outside of cells are decomposed by proteasome, bind to MHC class I molecules, and are presented on the cell surface. As to cross presentation, an HSP90-peptide complex has been demonstrated to be effective, making it possible to stimulate CD8⁺ T cells without using an adjuvant (non-patent documents 8 and 9). However, since stimulation of CD8⁺ T cells requires a large amount of the HSP90-peptide complex, there has been a demand for a means of efficiently stimulating CD8⁺ T cells.

### Prior Art Documents

### non-patent document

non-patent document 1: Lindquist S. Annu Rev Biochem. 1986, 55:1151-1191.
non-patent document 2: Morimoto RI, Santoro MG. Nat Biotechnol. 1998, 16:833-838.
non-patent document 3: Sidera K, Gaitanou M, Stellas D, Matsas R, Patsavoudi E. J Biol Chem. 2008, 283:2031-2041.
non-patent document 4: Tsutsumi S, Scroggins B, Koga F, et al. Oncogene. 2008, 27:2478-2487.
non-patent document 5: Sidera K, Samiotaki M, Yfanti E, Panayotou G, Patsavoudi E. J Biol Chem. 2004, 279:45379-45388.
non-patent document 6: Cheng CF, Fan J, Fedesco M, et al. Mol Cell Biol. 2008, 28:3344-3358.
non-patent document 7: Woodley DT, Fan J, Cheng CF, et al. J Cell Sci. 2009, 122:1495-1498.
non-patent document 8: Binder RJ, Srivastava PK. Nat Immunol. 2005, 6:593-599.
non-patent document 9: Kurotaki T, Tamura Y, Ueda G, et al. J Immunol. 2007, 179:1803-1813.

### Summary of the Invention

### Problems to Be Solved by the Invention

It is an object of the present invention to provide a novel anti-HSP90 antibody and use thereof, more specifically to provide a monoclonal antibody capable of recognizing HSP90 expressed on the cell surface, a method of cell identification using the antibody, a vaccine composed of the antibody and an antigen, a drug that utilizes the antibody, and use as an adjuvant.

### Means of Solving the Problem

The present inventors conducted extensive investigations in view of the above-described problems. As a result, the present inventors obtained a novel anti-HSP90 monoclonal antibody capable of recognizing HSP90 expressed on the cell surface with high sensitivity, and succeeded in identifying an epitope for the antibody by epitope mapping using a library which consists of peptides having overlapping amino acid sequences from HSP90.

The present inventors also found that rapid internalization of cell surface HSP90 is induced, and concurrently the antibody enters into the cells, as a result of binding of the antibody to cell surface HSP90 on cells expressing cell surface HSP90 (e.g., antigen-presenting cells or the like). Furthermore, the present inventors found that an antigen coupled to the antibody enters into antigen-presenting cells, and is then delivered to the MHC class I antigen presentation pathway, resulting in the activation of CD8⁺ T cells. The present inventors further found that the antibody has an adjuvant effect to enhance the antigen presentation mechanism via an Fc receptor. With these findings, the present inventors have completed the present invention.

Accordingly, the present invention provides:
[1] A monoclonal antibody that binds to an epitope comprising an amino acid sequence selected from the amino acid sequence VX₁X₂EX₃PPLEGDX₄ (wherein each of X₁ to X₄, which may be identical to or different from each other, represents an arbitrary amino acid) (SEQ ID NO:1) or the amino acid sequence HX₅IX₆ETLRQKAE (wherein each of X₅ to X₆, which may be identical to or different from each other, represents an arbitrary amino acid) (SEQ ID NO:2), and that recognizes cell surface HSP90.
[2] The monoclonal antibody according to [1], wherein X₂ in the amino acid sequence of SEQ ID NO:1 is E or D.
[3] The monoclonal antibody according to [1], wherein X₄ in the amino acid sequence of SEQ ID NO:1 is E or D.
[4] The monoclonal antibody according to [1], wherein X₆ in the amino acid sequence of SEQ ID NO:2 is I or V.
[5] The monoclonal antibody according to [1], wherein the epitope is an epitope comprising:
   (1) the amino acid sequence of SEQ ID NO:1 wherein X₁ is T, X₂ is E, X₃ is M, and X₄ is D,
   (2) the amino acid sequence of SEQ ID NO:1 wherein X₁ is P, X₂ is D, X₃ is I, and X₄ is E,
   (3) the amino acid sequence of SEQ ID NO:2 wherein X₅ is S, and X₆ is I, or
   (4) the amino acid sequence of SEQ ID NO:2 wherein X₅ is P, and X₆ is V.
[6] The monoclonal antibody according to any of [1] to [5], wherein the cell is an antigen-presenting cell.
[7] The monoclonal antibody according to [6], wherein the antigen-presenting cell is an activated dendritic cell, a macrophage or a monocyte (mononuclear leukocyte).
[8] The monoclonal antibody according to any of [1] to [5], wherein the cell is a tumor cell.
[9] The monoclonal antibody according to [8], wherein the tumor cell is of human origin.
[10] A hybridoma having an accession number of FERM BP-11222 or FERM BP-11243.
[11] A monoclonal antibody that binds to the same epitope as the epitope to which a monoclonal antibody produced by the hybridoma according to [10] binds, and that recognizes cell surface HSP90.
[12] The monoclonal antibody according to any of [1] to [9], which is produced by the hybridoma according to [10].
[13] An antibody that recognizes cell surface HSP90, comprising at least one of heavy-chain CDR1 (the amino acid sequence shown by the positions 66 to 70 of SEQ ID NO:10), CDR2 (the amino acid sequence shown by the positions 85 to 101 of SEQ ID NO:10), CDR3 (the amino acid sequence shown by the positions 134 to 141 of SEQ ID NO:10), light-chain CDR1 (the amino acid sequence shown by the positions 43 to 58 of SEQ ID NO:12), CDR2 (the amino acid sequence shown by the positions 74 to 80 of SEQ ID NO:12), and CDR3 (the amino acid sequence shown by the positions 113 to 121 of SEQ ID NO:12).
[14] An antibody that recognizes cell surface HSP90, comprising a heavy-chain variable region that comprises the amino acid sequence shown by the positions 36 to 185 of SEQ ID NO:10 and/or a light-chain variable region that comprises the amino acid sequence shown by the positions 20 to 177 of SEQ ID NO:12.
[15] A method of producing a monoclonal antibody that recognizes cell surface HSP90, comprising the step of administering to an animal a polypeptide as an immunogen that comprises as an epitope an amino acid sequence selected from the amino acid sequence VX₁X₂EX₃PPLEGDX₄ (wherein each of X₁ to X₄, which may be identical to or different from each other, represents an arbitrary amino acid) (SEQ ID NO:1) or the amino acid sequence HX₅IX₆ETLRQKAE (wherein each of X₅ to X₆, which may be identical to or different from each other, represents an arbitrary amino acid) (SEQ ID NO:2).
[16] A method of producing an antibody that recognizes cell surface HSP90, comprising the step of preparing a polypeptide that comprises at least one of heavy-chain CDR1 (the amino acid sequence shown by the positions 66 to 70 of SEQ ID NO:10), CDR2 (the amino acid sequence shown by the positions 85 to 101 of SEQ ID NO:10), CDR3 (the amino acid sequence shown by the positions 134 to 141 of SEQ ID NO:10), light-chain CDR1 (the amino acid sequence shown by the positions 43 to 58 of SEQ ID NO:12), CDR2 (the amino acid sequence shown by the positions 74 to 80 of SEQ ID NO:12), and CDR3 (the amino acid sequence shown by the positions 113 to 121 of SEQ ID NO:12).
[17] A vaccine comprising a complex of an antibody that recognizes HSP90 on the cell surface and a target antigen.
[18] The vaccine according to [17], wherein the antibody that recognizes HSP90 on the cell surface is the antibody according to any of [1] to [9] and [11] to [14].
[19] The vaccine according to [17] or [18], wherein the target antigen is a pathogen antigen or a tumor antigen.
[20] A method of producing a vaccine, comprising the step of forming a complex of an antibody that recognizes HSP90 on the cell surface and a target antigen.
[21] A method of inducing immunity, comprising the step of administering an effective amount of the vaccine according to any of [17] to [19] to a subject in need thereof.
[22] A method of treating or preventing a disease, comprising the step of administering an effective amount of the vaccine according to any of [17] to [19] to a subject in need thereof.
[23] A drug comprising a complex of an antibody that recognizes HSP90 on the cell surface and a substance possessing a biological activity.
[24] The drug according to [23], wherein the antibody that recognizes HSP90 on the cell surface is the antibody according to any of [1] to [9] and [11] to [14].
[25] The drug according to [23] or [24], wherein the substance possessing a biological activity is a nucleic acid molecule.
[26] The drug according to [25], wherein the nucleic acid molecule is an siRNA.
[27] The drug according to [23] or [24], wherein the substance possessing a biological activity is an anticancer agent.
[28] A method of treating or preventing a disease, comprising administering an effective amount of the drug according to any of [23] to [27] to a subject in need thereof.
[29] An adjuvant comprising a polypeptide that comprises the antigen-binding site of an antibody that recognizes HSP90 on the cell surface.
[30] The adjuvant according to [29], wherein the antibody that recognizes HSP90 on the cell surface is the antibody according to any of [1] to [9] and [11] to [14].
[31] The adjuvant according to [29], wherein the antibody that recognizes HSP90 on the cell surface recognizes the amino acid sequence of SEQ ID NO:3 (VTEEMPPLEGDD) as an epitope.
[32] The adjuvant according to [29], wherein the antigen-binding site of the antibody that recognizes HSP90 on the cell surface comprises the light-chain variable region in the antigen-binding site of the monoclonal antibody produced by the hybridoma whose accession number is FERM BP-11222.
[33] A method of producing an adjuvant, comprising the step of mixing a polypeptide that comprises the antigen-binding site of an antibody that recognizes HSP90 on the cell surface with a pharmaceutically acceptable excipient or additive.
[34] A method of inducing immunity, comprising the step of administering a vaccine that is incorporated into an antigen-presenting cell via binding to an Fc receptor and the adjuvant according to any of [29] to [32] to a subject in need thereof.
[35] The method of inducing immunity according to [34], wherein the antigen-presenting cell is a dendritic cell.
[36] The method of inducing immunity according to [34], wherein the vaccine is the vaccine according to any of [17] to [19].
[37] The method of inducing immunity according to [34], wherein the vaccine comprises a complex of a target antigen and an antibody.
[38] The method of inducing immunity according to [37], wherein the target antigen is a pathogen antigen or a tumor antigen.
[39] A method of treating or preventing a disease, comprising the step of administering an effective amount of a vaccine that is incorporated into an antigen-presenting cell via binding to an Fc receptor and an effective amount of the adjuvant according to any of [29] to [32] to a subject in need thereof.
[40] A method of inducing immunity, comprising the step of administering an anti-tumor antigen antibody and the adjuvant according to any of [29] to [32] to a subject in need thereof.
[41] A method of inducing immunity, comprising the step of administering an anti-tumor cell antibody and the adjuvant according to any of [29] to [32] to a subject in need thereof.
[42] A method of inducing immunity, comprising the step of administering an anticancer agent and the adjuvant according to any of [29] to [32] to a subject in need thereof.
[43] A method of treating or preventing a disease, comprising the step of administering an effective amount of an anti-tumor antigen antibody and an effective amount of the adjuvant according to any of [29] to [32] to a subject.
[44] A method of detecting a cell expressing HSP90 on the cell surface, comprising:
   (i) the step of reacting the antibody according to any of [1] to [9] and [11] to [14] with a cell not subjected to permeabilization, and,
   (ii) the step of assessing the presence or absence of the formation of a complex of the antibody and HSP90 expressed on the cell membrane.
[45] The method according to [44], wherein a flow cytometer is used in the step of assessing the presence or absence of the formation of the complex.
[46] The method according to [44], wherein the cell expressing HSP90 on the cell surface is an antigen-presenting cell.
[47] The method according to [46], wherein the antigen-presenting cell is an activated dendritic cell, a macrophage or a monocyte (mononuclear leukocyte).
[48] The method according to [44], wherein the cell expressing HSP90 on the cell surface is a tumor cell.
[49] The method according to [48], wherein the tumor cell is of human origin.

### Effect of the Invention

The anti-HSP90 antibody of the present invention is capable of recognizing cell surface HSP90 with high sensitivity, and hence is capable of detecting cell surface HSP90 of living cell in FACS analysis. Because of its capability to specifically recognize cell surface HSP90 with high sensitivity, the antibody of the present invention can be used, for example, for preparing the vaccine of the present invention, for delivering a substance possessing a biological activity, for an adjuvant that enhances a function of a vaccine, a tumor antigen antibody or an anti-tumor cell antibody, and in a method of specifically detecting a cell expressing cell surface HSP90.

A vaccine composed of the anti-HSP90 antibody provided by the present invention and an antigen is capable of delivering the antigen only to antigen-presenting cells, particularly to activated dendritic cells. Because of its capability to efficiently activate T cells with a small amount of antigen, the vaccine can be used to treat a wide variety of diseases by choosing the antigen appropriately.

Because of its capability to specifically recognize HSP90 on the cell surface and to quickly enter into cells after binding thereto, the anti-HSP90 antibody provided by the present invention makes it possible to efficiently deliver a substance possessing a biological activity to a cell expressing HSP90 on the cell surface.

The adjuvant provided by the present invention is capable of enhancing the antigen presentation mechanism in dendritic cells and the like that occurs upon binding to an Fc receptor, via binding to a complex composed of HSP90 and FcRγ on the cell surface. For example, when used in combination with a vaccine, the adjuvant provided by the present invention makes it possible to reduce the amount of the vaccine used, thus reducing the potential risk of adverse reactions.

Furthermore, a cell expressing HSP90 on the cell surface in the form of a living cell, which has conventionally been undetectable, can be detected according to the present invention. Thus, the present invention can be used to, for example, detect, isolate, and analyze antigen-presenting cells.

### Brief Description of the Drawings

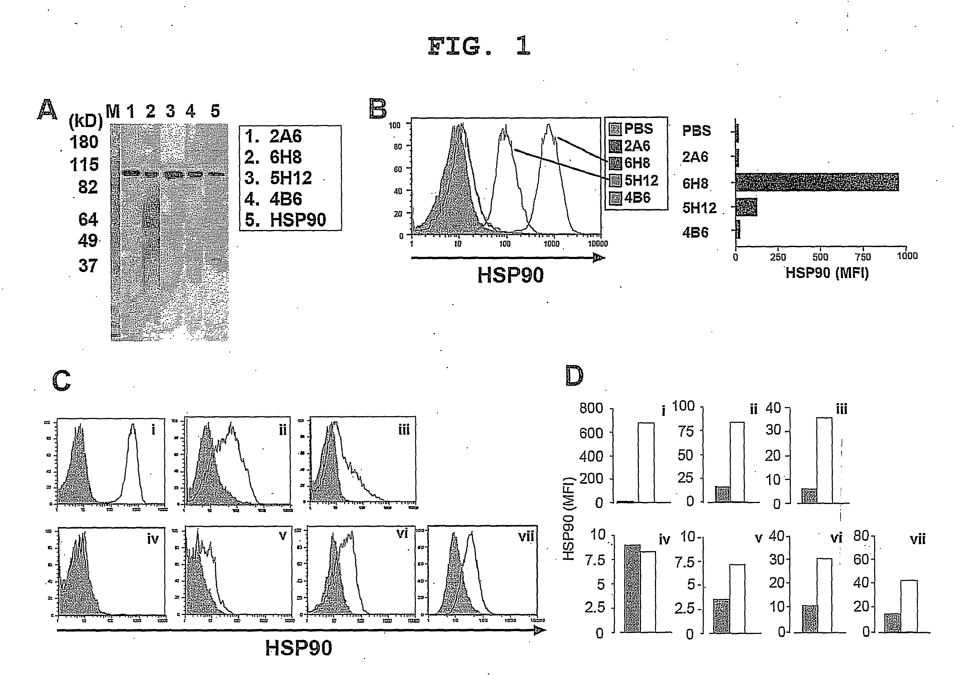

Fig. 1A shows the results of Western blot analyses using monoclonal antibodies. A DC2.4 dendritic cell line extract was developed on SDS-PAGE and then blotted using the indicated antibodies. All the monoclonal antibodies detected HSP90 as a single band. Fig. 1B shows the results of FACS analyses of cell surface HSP90 using monoclonal antibodies. Fig. 1B shows FACS data on DC2.4 cells stained using PE-cy5 streptavidin and the respective biotinylated anti-HSP90 monoclonal antibodies. Figs. 1C and D show the results of FACS analyses of antigen-presenting cells using the 6H8 antibody.

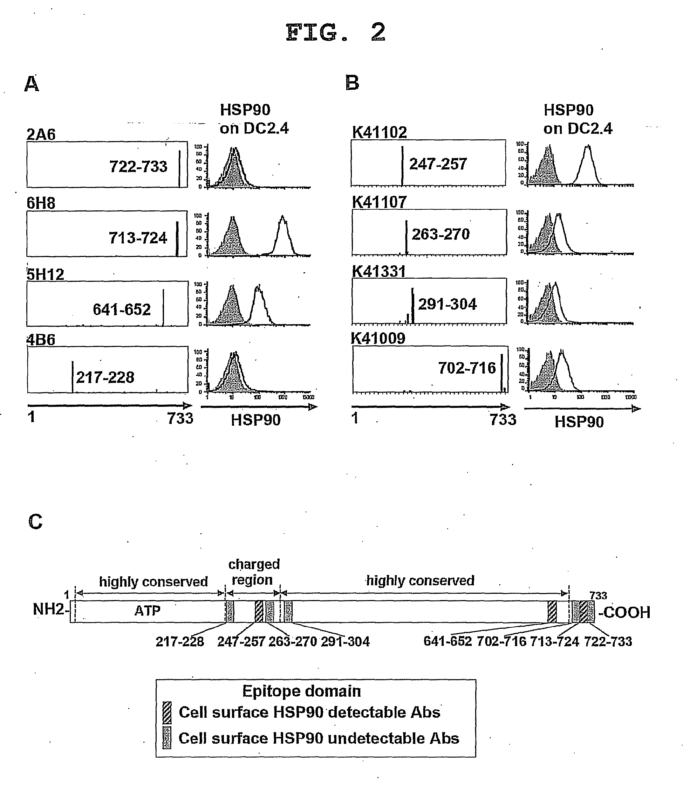

Fig. 2 shows the results of epitope mapping of anti-HSP90 monoclonal antibodies. Figs. 2A and B show relative values of the results of an ELISA assay using respective peptides in a synthetic peptide library that consists of peptides corresponding to the sequences on HSP90α (left panel), and FACS data on DC2.4 cells stained with the respective antibodies (histogram, right panel). Fig. 2C schematically summarizes the results of the epitope mapping.

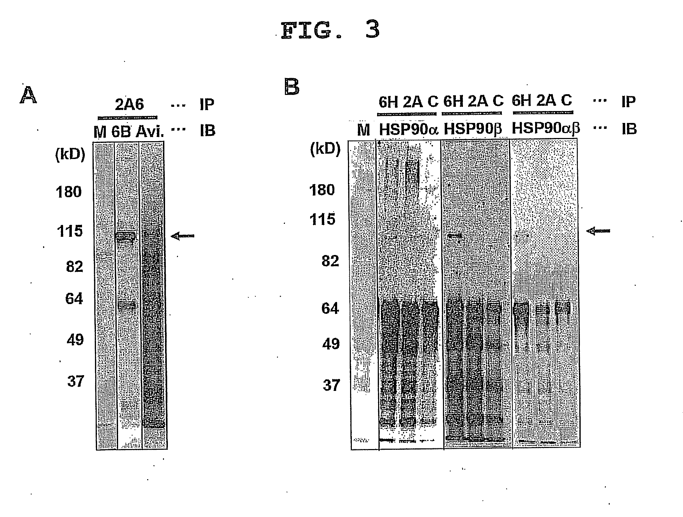

Fig. 3 shows the results of immunoprecipitation of cell surface HSP90 with the 6H8 antibody. Fig. 3A shows the detection of biotinylated HSP90 in a membrane fraction prepared from DC2.4 cells which had been biotinylated on the cell surface. It is shown that HSP90 immunoprecipitated with the 2A6E9 antibody from the cell membrane fraction (lane 6B) is biotinylated (lane Avi.). Fig. 3B shows the detection of cell surface HSP90α and β immunoprecipitated with the 6H8 antibody.

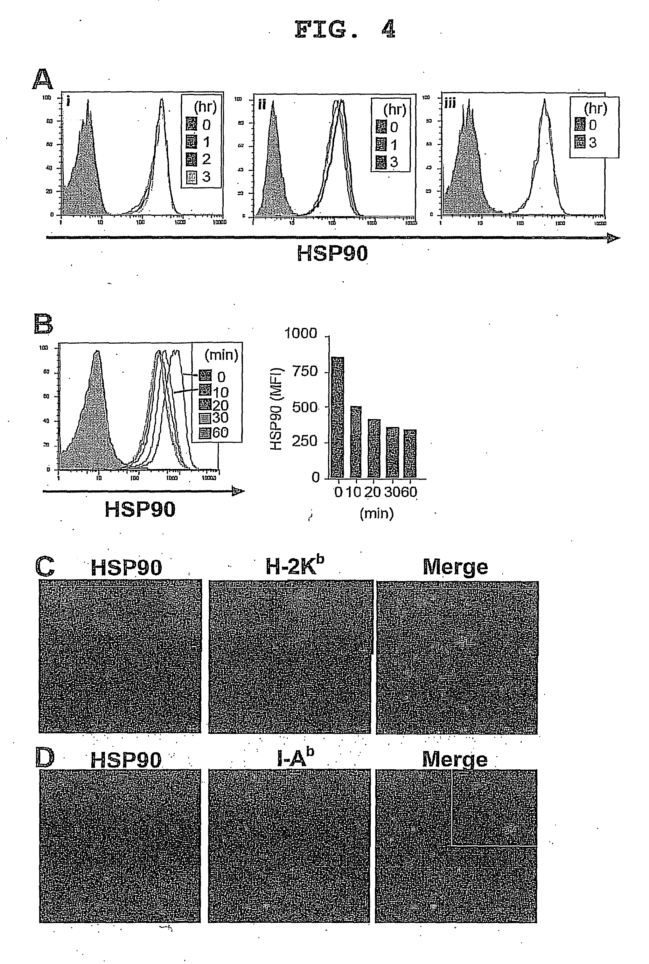

Fig. 4 demonstrates the internalization of cell surface HSP90 in DC2.4 cells by the 6H8 antibody. Fig. 4A shows that OVA (i) and the HSP90 inhibitors radicicol (ii) and novobiocin (iii) do not influence the status of occurrence of cell surface HSP90. Fig. 4B shows the results obtained by binding the biotinylated 6H8 antibody to cell surface HSP90 on DC2.4 cells at 4°C, washing away the unbound antibody, then increasing the temperature to 37°C, returning the temperature to 4°C over time (0, 10, 20, 30, 60 minutes), labeling the cells with PE-Cy5-streptavidin, and detecting and quantifying over time the 6H8 antibody present on the cell surface. Fig. 4C shows the localization of HSP90 and H-2K^{b} in DC2.4 cells at 20 minutes in Fig. 4B. Fig. 4D shows the localization of HSP90 and I-A^{b} in DC2.4 cells. The HSP90 which was initially present on the cell membrane surface mostly entered into the cells, whereas H-2K^{b} or I-A^{b} remained on the cell surface.

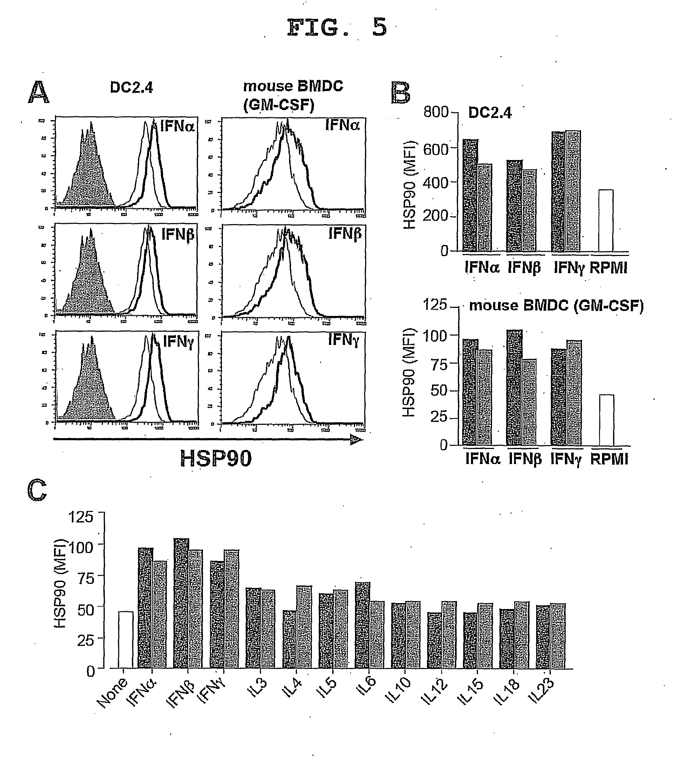

Fig. 5 shows the influences of cytokines on the expression level of cell surface HSP90. Figs. 5A and B show the results of FACS analyses of changes in the expression of cell surface HSP90 in DC2.4 cells and mouse BMDC after treatment with IFNα, β or γ. Fig. 5C shows the results of FACS analyses of changes in the expression of cell surface HSP90 in BMDC after treatment with various cytokines. Solid bars: 100 ng/ml (IFNα, β: 500 U/ml, IFNγ: 50 U/ml); grey bars: 10 ng/ml (IFNα, β: 100 U/ml, IFNγ: 10 U/ml)

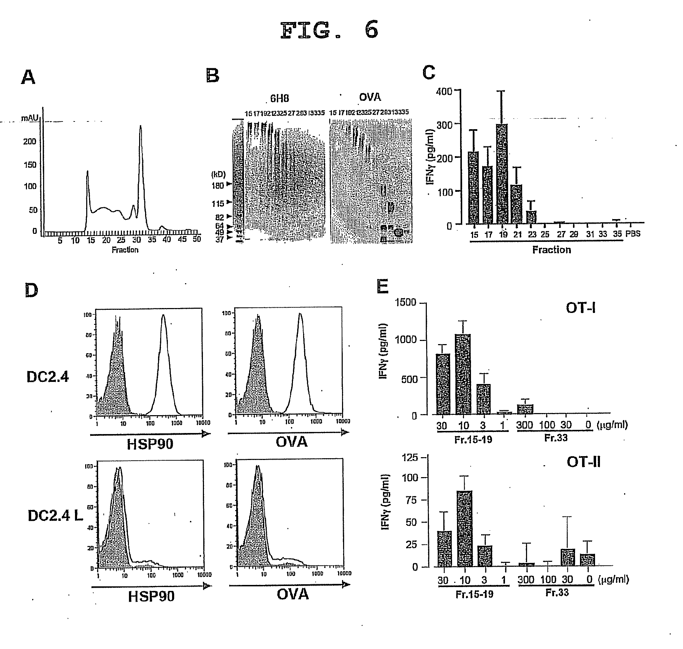

Fig. 6 shows the results of a cross-presentation assay using a 6H8-OVA conjugate. Fig. 6A shows a size exclusion chromatogram of a reaction mixture of the 6H8 antibody and maleimidated OVA. Fig. 6B shows the presence or absence and the molecular weight profiles of the 6H8 antibody and OVA in each fraction. Fig. 6C shows the induction of IFNγ production from OT-I CD8⁺ T cells in DC2.4 cells treated with each fraction. Fig. 6D shows the results of FACS analyses showing the recognition of HSP90 on the surface of DC2.4 and DC2.4L (cell surface HSP90-deficient mutant line) cells (left panel). At the same time, the cells were stained with an anti-OVA antibody after reacting with the 6H8-OVA conjugate (right panel). As expected, the results showed that the cell surface HSP90-deficient mutant line DC2.4L did not become stained with the anti-OVA antibody. Fig. 6E shows the induction of IFNγ production from OT-I CD8⁺ T cells and OT-II CD4⁺ T cells when DC2.4 was treated with a dilution of the 6H8-OVA conjugate or free OVA (fraction 33).

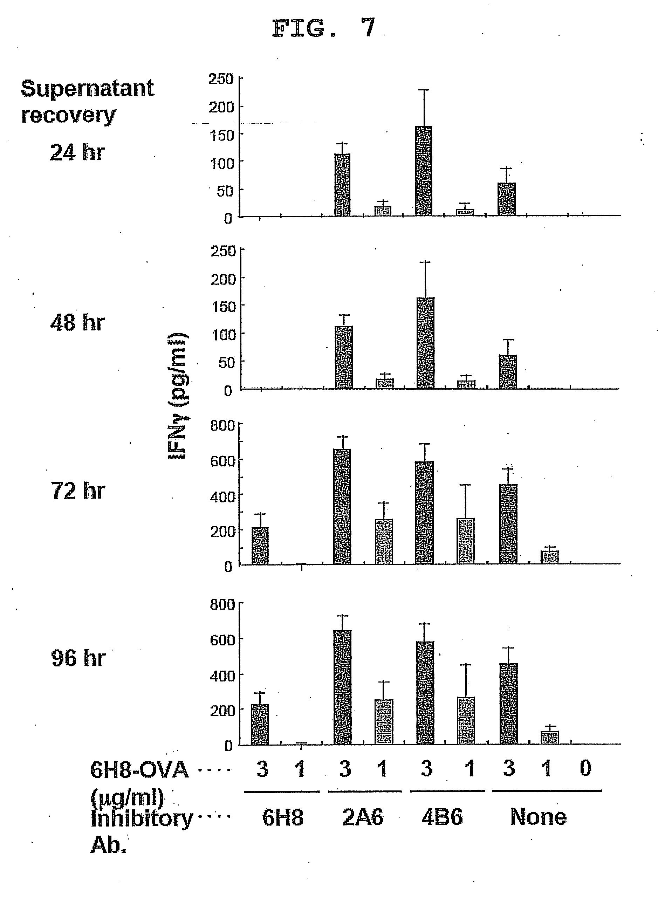

Fig. 7 shows the inhibition of the immunological effect of the 6H8-OVA conjugate by the 6H8 antibody in a cross-presentation assay in vitro.

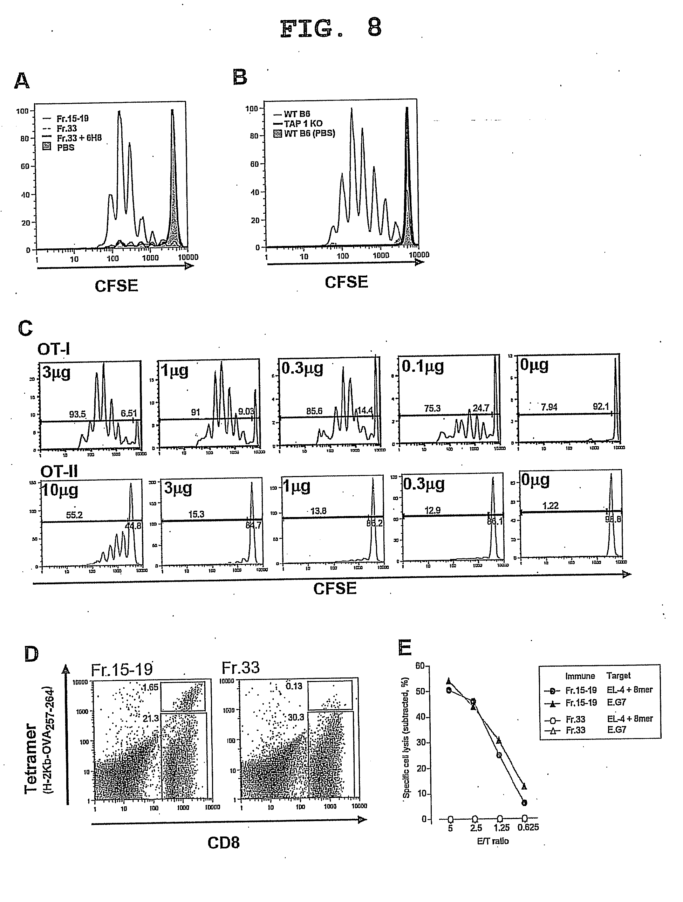

Fig. 8A shows the induction of proliferation of OT-I CD8⁺ T cells by the 6H8-OVA conjugate in vivo. Fig. 8B shows the lack of proliferation of OT-I CD8⁺ T cells by the 6H8-OVA conjugate in vivo observed when using a TAP1-deficient mouse. Fig. 8C shows the induction of proliferation of OT-I CD8⁺T cells and OT-II CD4⁺ T cells by a series of doses of the 6H8-OVA conjugate in vivo. Figs. 8D and E show the results obtained by immunizing mice with the 6H8-OVA conjugate, attempting to induce cytotoxic T cells from the splenocytes thereof.

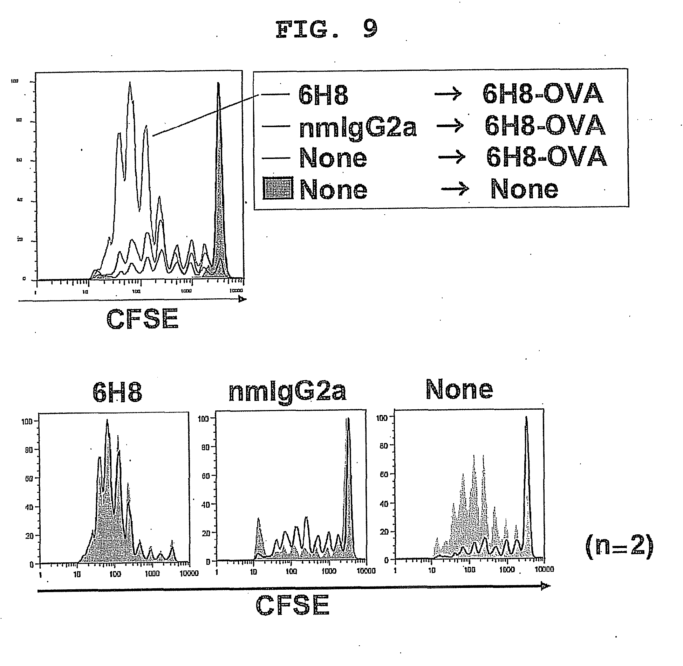

Fig. 9 shows that the 6H8 antibody enhances the induction of proliferation of OT-I CDB⁺T cells by the 6H8-OVA conjugate in vivo.

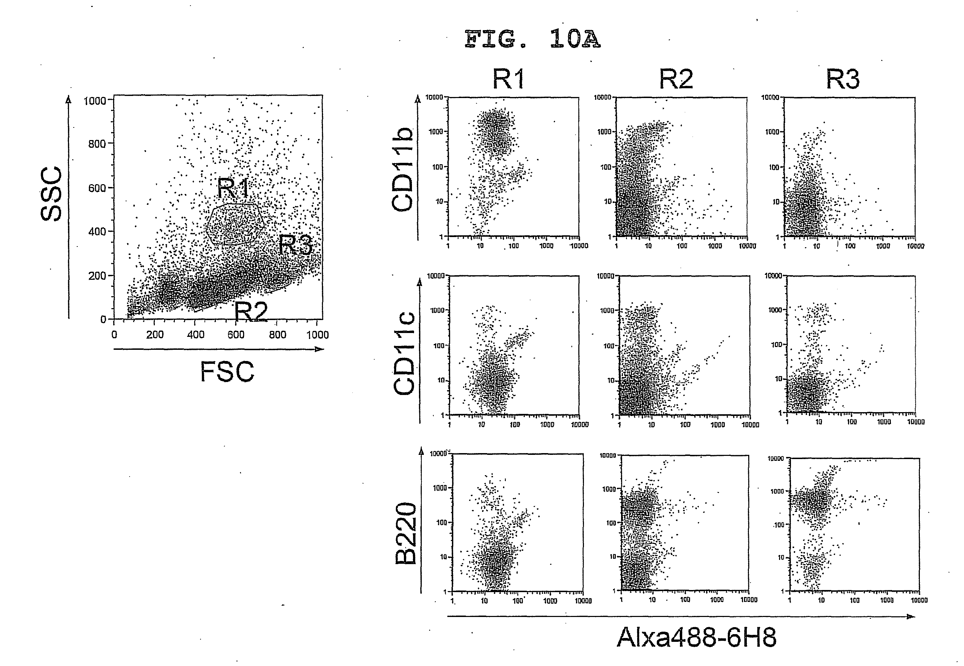

Fig. 10A shows that the Alexa 488-labeled 6H8 antibody administered to mice accumulates in CD11b⁺ monocytes in the spleen. The Alexa 488-labeled 6H8 antibody was administered to B6 mice; 3 hours later, the spleen was recovered from each mouse and treated with collagenase. The resulting sample was analyzed for the expression of CD11b, CD11c and B220 by FACS.

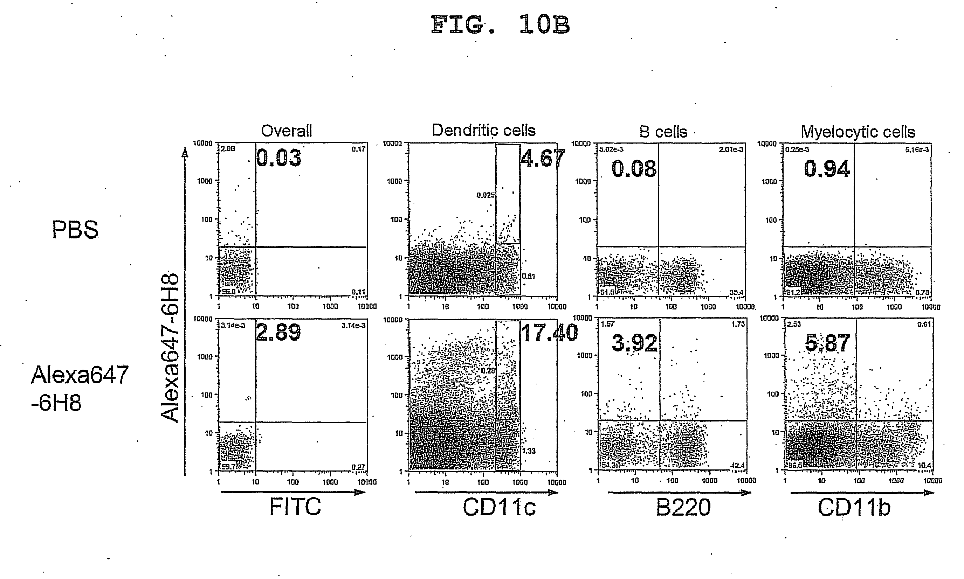

Fig. 10B shows that the Alexa 647-labeled 6H8 antibody administered to mice accumulates in dendritic cells (CD11c-positive) in the spleen. The Alexa 647-labeled 6H8 antibody was administered to B6 mice; 3 hours later, the spleen was recovered from each mouse and treated with collagenase. The resulting sample was analyzed for the expression of CD11b CD11c, and B220 by FACS, and the accumulation of the labeled 6H8 antibody in various cells was compared. Shown are the results for a region corresponding to the R2 region in Fig. 10A.

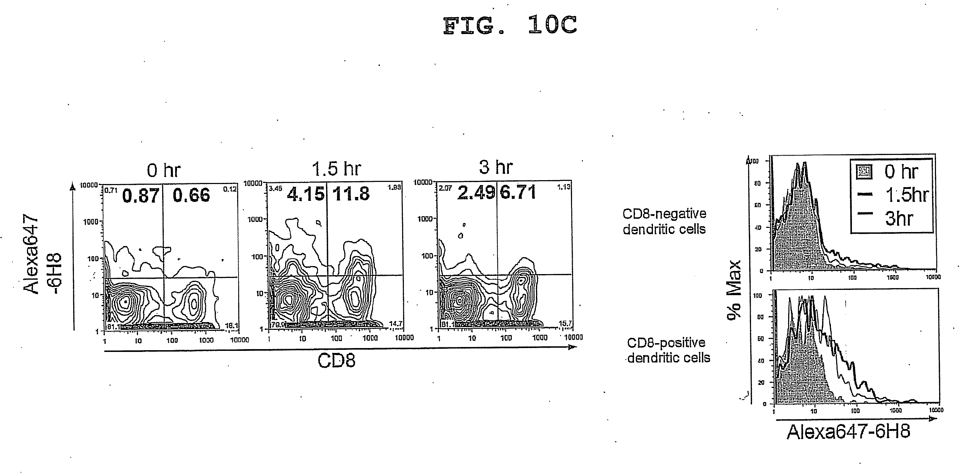

Fig. 10C shows that the Alexa 647-labeled 6H8 antibody administered to mice accumulates in CD8α-positive dendritic cells in the spleen. For samples prepared in the same manner as in Fig. 10B except that the spleen was recovered 0, 1.5, or 3 hours after administration of the Alexa 647-labeled 6H8 antibody, the expression of CD8 was analyzed by FACS, and the accumulation of the labeled 6H8 antibody in dendritic cells was examined.

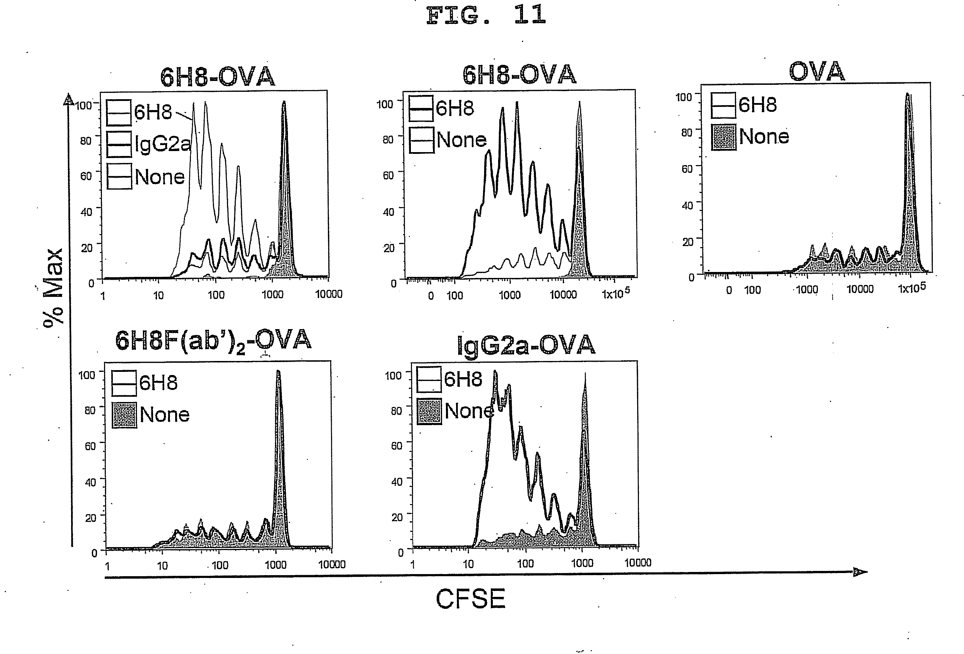

Fig. 11 shows that 6H8-OVA promotes Fc receptor-dependent cross-presentation in vivo. CFSE-labeled CD8⁺ T cells of a OT-I/CD45.1 mouse were transferred to recipient mice from the tail vein, and 6H8-OVA [or 6H8 (F(ab')₂)-OVA, or OVA, or IgG2a-OVA] and the free 6H8 antibody [or 6H8 (F(ab')₂) or IgG2a] (30 µg) were administered concurrently. 72 hours later, the spleen was recovered from each mouse, and the splenocytes were stained with the PE-CD45.1 antibody, after which the CFSE intensity of CD45.1-positive cells (OT-1 CD8⁺ T cells) was determined.

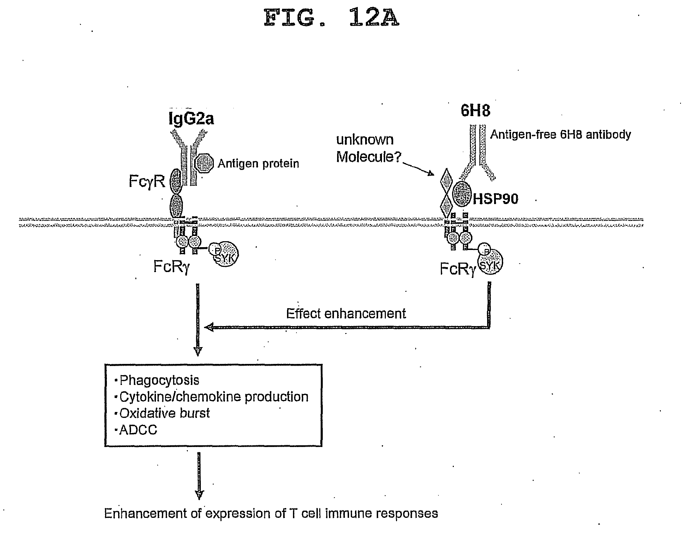

Fig. 12A illustrates the mechanism by which the 6H8 antibody functions as an adjuvant. Shown is a case where IgG2a conjugated with an antigen protein was used as a vaccine. The vaccine binds to an Fcγ receptor (FcγR) via the Fc portion of IgG2a, whereby the phosphorylation of SYK by FcRγ present inside of the cell membrane occurs, causing many immune responses. Meanwhile, the antigen-binding site of 6H8 is thought to cause the phosphorylation of SYK via FcRγ in a protein complex comprising cell surface HSP90 by binding to cell surface HSP90, whereby the 6H8 antibody functions as an adjuvant to enhance the signal of the vaccine and hence enhance immunoreactions.

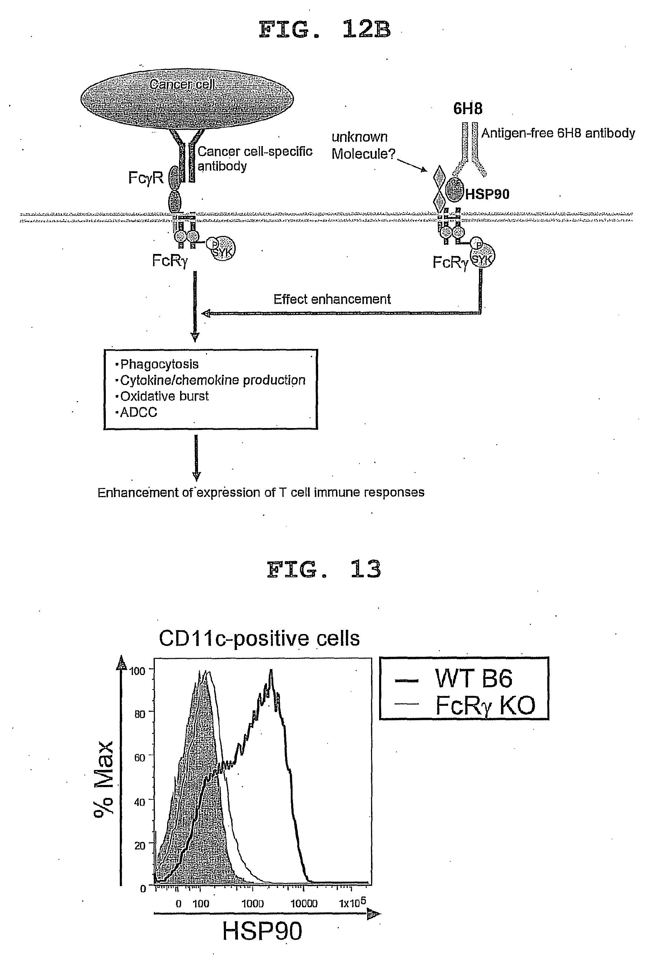

Fig. 12B illustrates the mechanism by which the 6H8 antibody functions as an adjuvant. Shown is a case where an anti-tumor cell antibody (cancer cell-specific antibody) was used as a vaccine. Tumor cells (or fragments thereof) bound to such an antibody are incorporated into dendritic cells via an Fcγ receptor, causing many immune responses. These immune responses are amplified by binding of the antigen-binding site of 6H8 to cell surface HSP90.
Fig. 13 shows that the expression of cell surface HSP90 in dendritic cells ("HSP90" in the figure) is dependent on FcRγ. Dendritic cells (BMDC) were established from the bone marrows of a normal mouse and an FcRγ KO mouse and analyzed for the expression of cell surface HSP90.

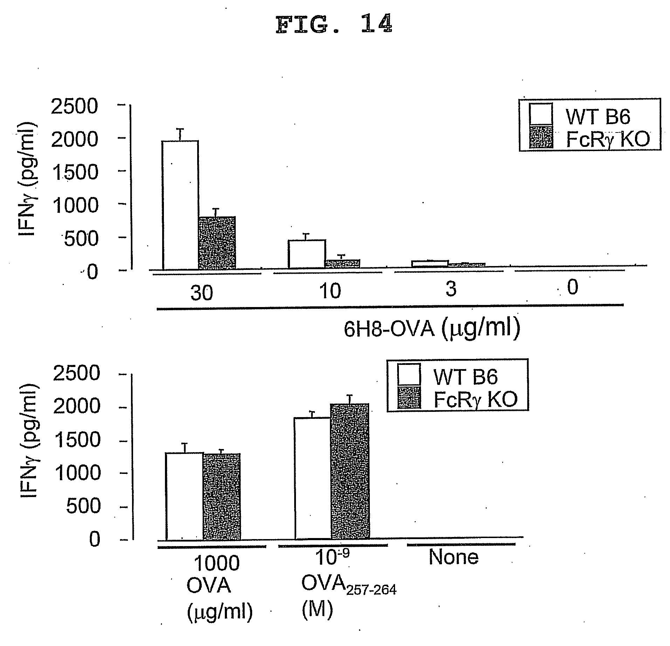

Fig. 14 shows that the cross-presentation potential of FcRγ KO mouse-derived dendritic cells (FcRγKO) for 6H8-OVA is significantly lower than that of normal mouse-derived dendritic cells (WT B6). An antigen (6H8-OVA, OVA, or OVA₂₅₇₋₂₆₄) was added to BMDC prepared in the same manner as in Fig. 13; after incubation for 3 hours, the cells were fixed. These cells and OT-I CD8⁺ T cells were co-cultured, and the amount of IFNγ produced in the culture supernatant was determined by ELISA.

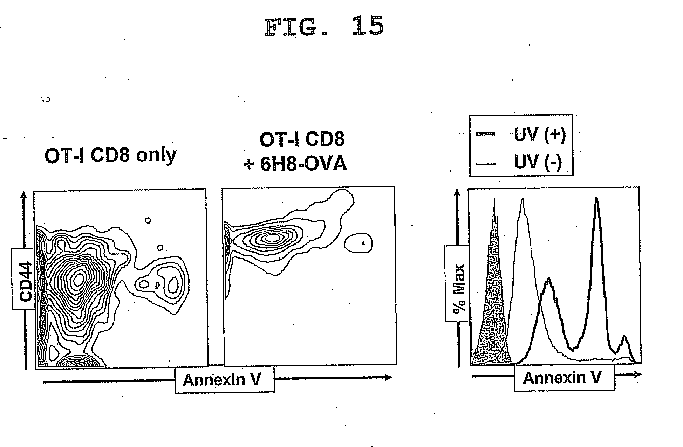

Fig. 15 shows that adoptively transferred OT-I CD8⁺ T cells are activated and differentiate into memory T cells by administration of 6H8-OVA in vivo. CD8⁺ cells of an OT-I/CD45.1 mouse were transferred to a C57BL/6 mouse, with 6H8-OVA or PBS administered concurrently. 96 hours later, the mouse spleen was recovered, and the splenocytes were analyzed for the expression of CD44 and CD45.1 (left panel). Also, cells that had undergone apoptosis were stained with Annexin V. The right panel shows the results of a control experiment confirming the function of Annexin V as a reagent.

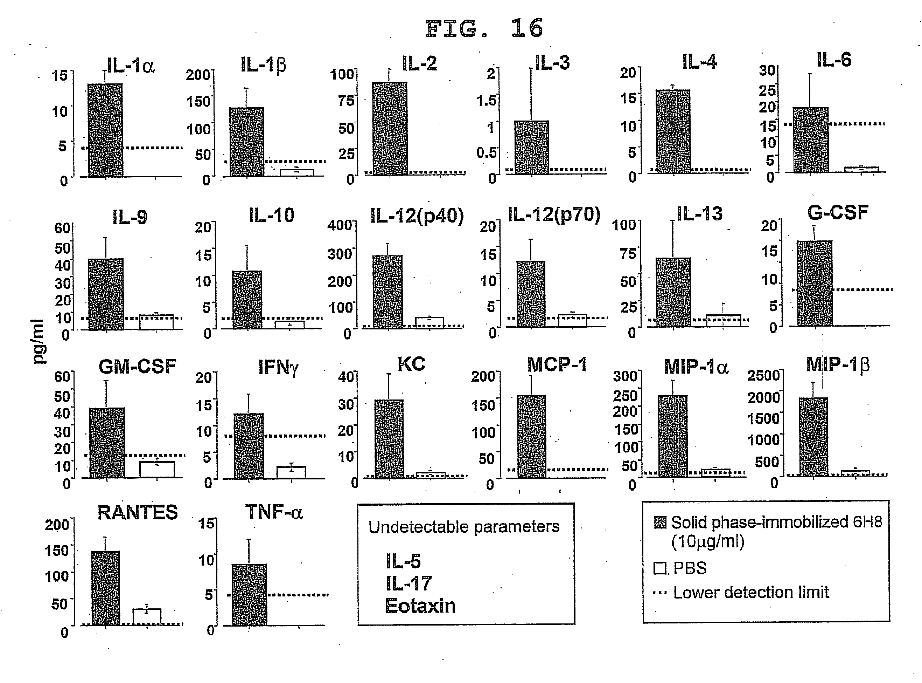

Fig. 16 shows the induction of cytokine production from bone marrow-derived dendritic cells by stimulation with the 6H8 antibody immobilized on a solid phase. GM-CSF-dependent bone marrow-derived dendritic cells were cultured for 12 hours in a 96-well flat plate with the 6H8 antibody immobilized thereon, and the amounts of cytokines and chemokines in the culture supernatant were measured. "PBS" shows the results of a control experiment performed using a plate without the 6H8 antibody immobilized thereon.

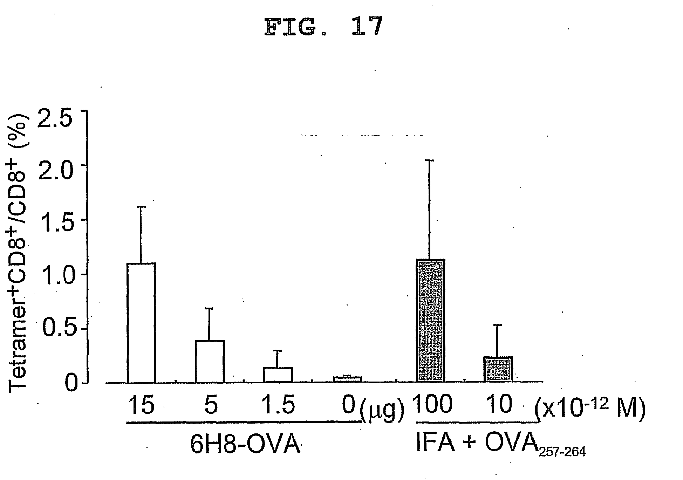

Fig. 17 shows that the efficiency of induction of specific CTL in the case where an antigen is conjugated to the 6H8 antibody is more than the efficiency in the case where the antigen is used along with a preexisting adjuvant. Examined was the induction of specific CTL in the splenocytes of mice immunized with 6H8-OVA or IFA+OVA₂₅₇₋₂₆₄. The panel shows the ratio of tetramer staining-positive cells in CD8-positive cells.

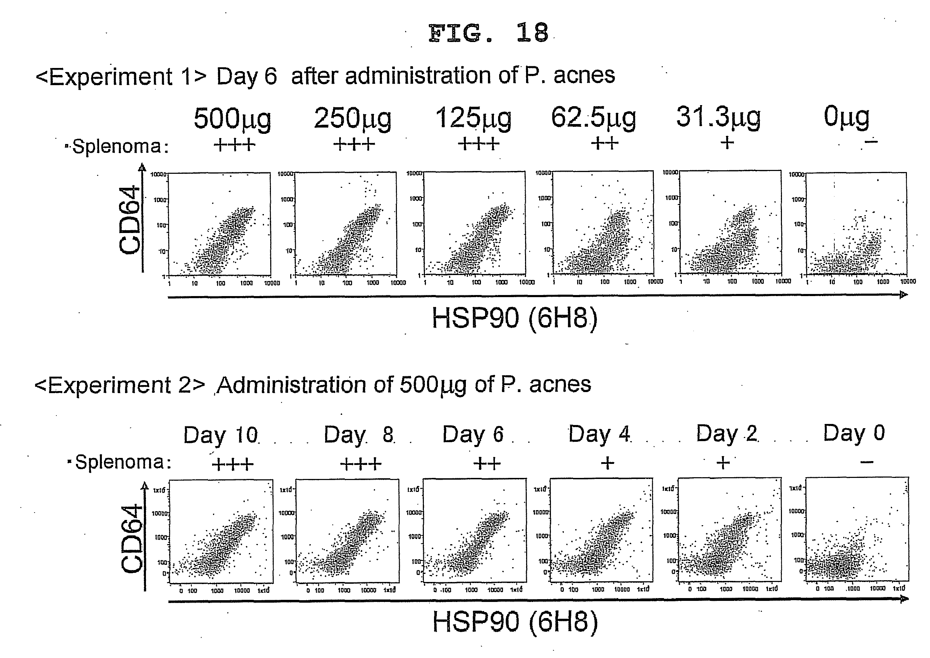

Fig. 18 shows that administration of P. acnes induces the expression of HSP90 and CD64 (Fcγ receptor) on the surface of spleen dendritic cell membrane. "Experiment 1" shows the results of analyses of the expression of HSP90 and CD64 in mouse splenocytes 6 days after administration of various amounts of P. acnes. "Experiment 2" shows the results of analyses of the expression of HSP90 and CD64 in mouse splenocytes 0 to 10 days after administration of 500 µg of P. acnes.

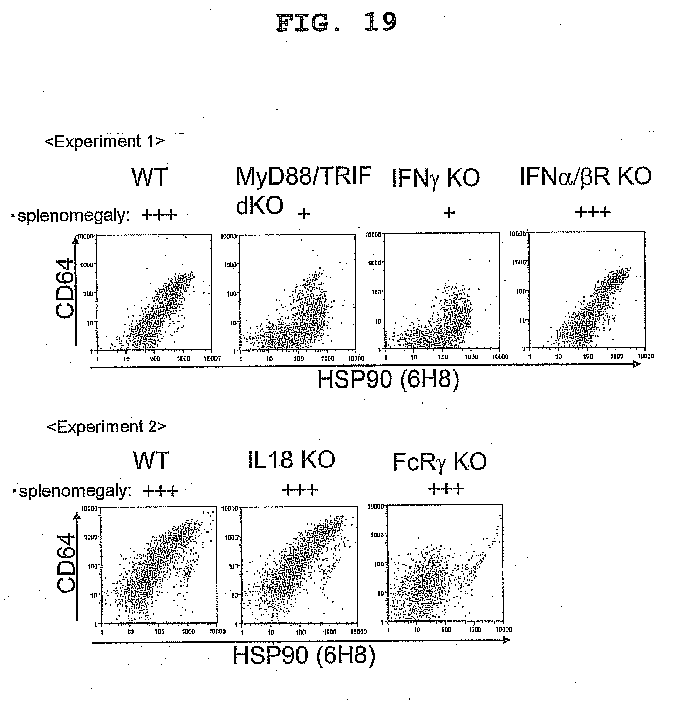

Fig. 19 shows that the expression of HSP90 and CD64 on the surface of spleen dendritic cell membrane by administration of P. acnes is dependent on FcRγ. Shown are the results of analyses of the expression of HSP90 and CD64 in mouse splenocytes 6 days after administration of 500 µg of P. acnes to wild-type mice and various types of knockout mice.

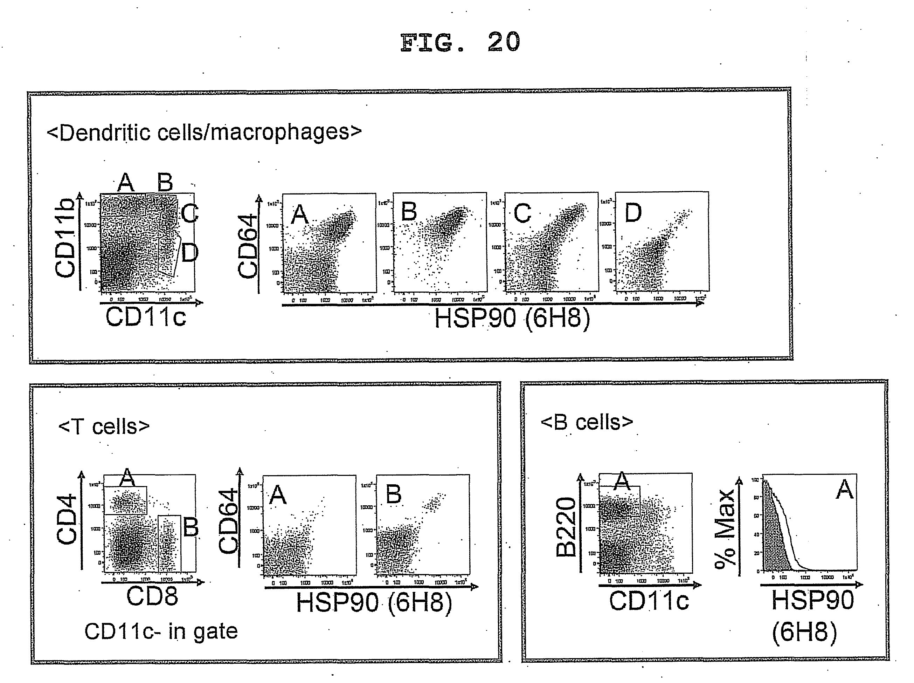

Fig. 20 shows that the expression of HSP90 on the cell membrane surface by administration of P. acnes is limited to antigen-presenting cells. Shown are the results of analyses of the expression of HSP90 and CD64 in mouse splenocytes 6 days after administration of 500 µg of P. acnes to wild-type mice.

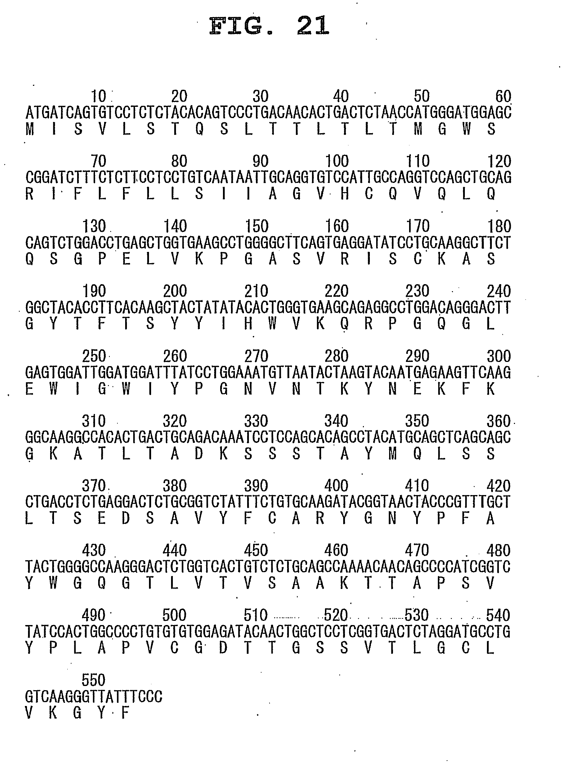

Fig. 21 shows the gene sequence and amino acid sequence of the heavy chain of the 6H8 monoclonal antibody.

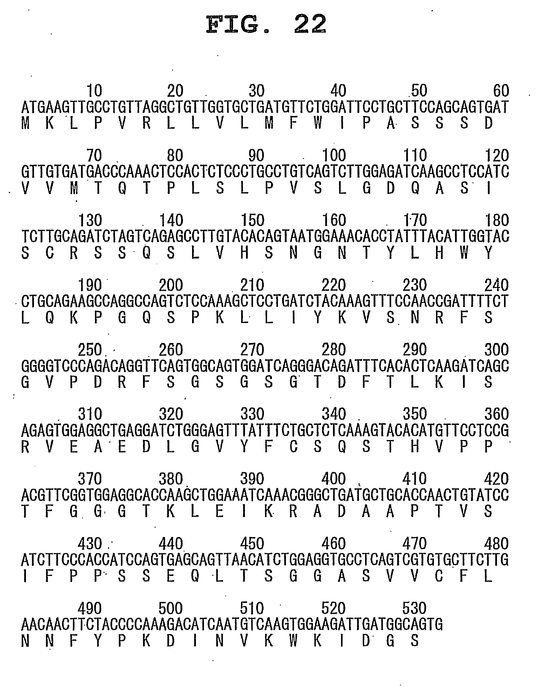

Fig. 22 shows the gene sequence and amino acid sequence of the light chain of the 6H8 monoclonal antibody.

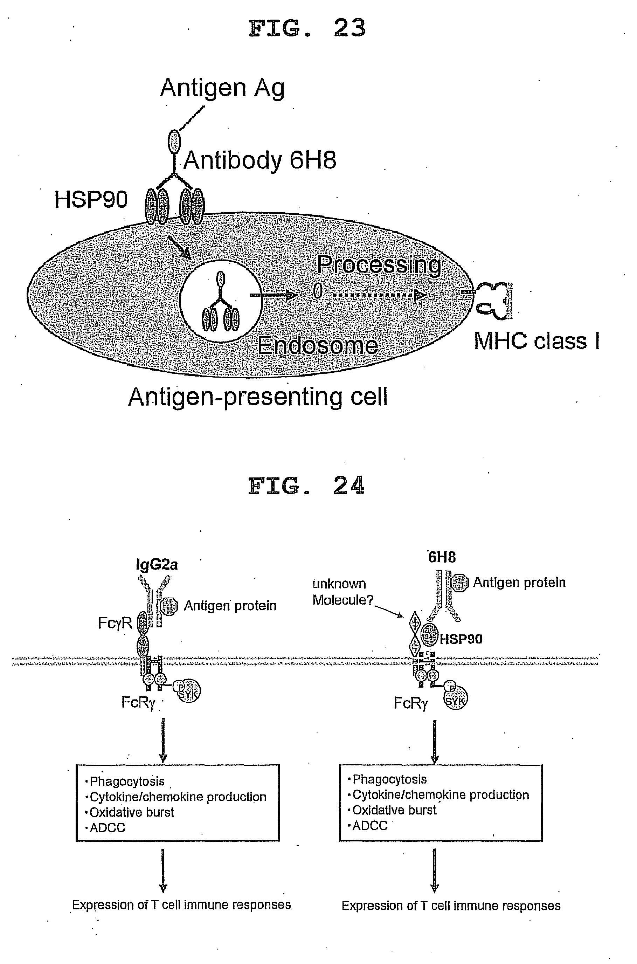

Fig. 23 illustrates the cross-presentation of an antigen conjugated to an antibody that recognizes cell membrane surface HSP90. The antibody, upon binding to HSP90 on an antigen-presenting cell, forms an antibody-HSP90 complex, which is then incorporated into the cell by endocytosis. The antigen conjugated to the antibody is delivered to the MHC class I antigen presentation pathway by the processing mechanism in the cell and presented to CD8⁺ T cells.
Fig. 24 illustrates that the IgG2a-antigen protein and the 6H8-antigen protein exhibit vaccine effects in a FcRy-dependent manner. IgG2a binds to an Fcγ receptor via the Fc portion thereof. Thereby, the phosphorylation of SYK by FcRγ present inside of the cell membrane occurs, causing many immune responses. Meanwhile, it is thought that the antigen-binding site of 6H8 binds to cell surface HSP90 to cause the phosphorylation of SYK via FcRγ in a protein complex comprising cell surface HSP90.

### Description of Embodiments

All technical terms and scientific words used herein have the same meanings as those that are generally understood by those skilled in the technical field to which the present invention belongs unless otherwise specified in the text. Any methods and materials that are similar or equivalent to those described herein can be used for carrying out or testing the present invention; preferred methods and materials are described below. The disclosures in all publications, patent applications and patents mentioned herein are incorporated herein by reference, for example, for the purpose of describing and disclosing the constructs and methodologies described therein that can be used in relation to the inventions described herein.

Herein, amino acids, peptides and proteins are denoted using the following abbreviations adopted by the IUPAC-IUB Commission on Biochemical Nomenclature (CBN). The sequences of the amino acid residues of peptides and proteins are shown in a way such that the N-terminus is at the left end and the C-terminus at the right end, unless otherwise specified.
Ala or A: alanine
Val or V: valine
Leu or L: leucine
Ile or I: isoleucine
Pro or P: proline
Phe or F: phenylalanine
Trp or W: tryptophan
Met or M: methionine
Gly or G: glycine
Ser or S: serine
Thr or T: threonine
Cys or C: cysteine
Gln or Q: glutamine
Asn or N: asparagine
Tyr or Y: tyrosine
Lys or K: lysine
Arg or R: arginine
His or H: histidine
Asp or D: aspartic acid
Glu or E: glutamic acid

The present invention provides an anti-HSP90 monoclonal antibody capable of recognizing cell surface HSP90 that recognizes a particular sequence on HSP90, a hybridoma that produces the antibody, a vaccine comprising a complex of the antibody and an antigen bound thereto, use thereof, a drug comprising a complex of the antibody and a compound possessing a biological activity bound thereto, an adjuvant comprising the antibody, use thereof, a method of detecting a cell expressing cell surface HSP90 using the antibody, and the like.

In the present invention, HSP90 means a group of proteins having a molecular weight of 90 kDa whose expression is increased upon exposure to a wide variety of stresses such as heat to protect cells. As such, HSP90 functions as a molecular chaperon. Two isoforms of HSP90, HSP90α and β, are known. Therefore, in the present invention, HSP90 means both of the isoforms HSP90α and β.

The amino acid sequences of HSP90α and β and the base sequences encoding the same are known for many mammals. For example, the base sequences and amino acid sequences of human HSP90α (NM_005348, NP_005339), mouse HSP90α (NM_010480, NP_034610), human HSP90β (NM_007355, NP_031381), and mouse HSP90β (NM_008302, NP_032328) have been published, respectively. The amino acid sequences of HSP90α and β are well conserved among different mammalian species; for example, comparing the human and mouse sequences, HSP90α and HSP90β are known to have sequence identities of 99.0% and 99.6%, respectively. The sequence homologies between HSP90α and β for human and mouse are reportedly 86.1% and 86.2%, respectively. Therefore, these sequences can exhibit 100% sequence identity in short regions (e.g., several tens of amino acids) even among different species or different isoforms. For example, as stated below, the sequence of the epitope for the monoclonal antibody of the present invention exhibits 100% identity between the mouse and the human.

In the present invention, cell surface HSP90 means HSP90 that is expressed in the vicinity of the cell membrane of a cell. Cell surface HSP90 has been reported to occur in antigen-presenting cells [e.g., activated dendritic cells, macrophages, monocytes (mononuclear leukocytes) and the like], multipotential mesenchymal precursor cells (MPC) (Gronthos S, McCarty R, Mrozik K, et al., Stem Cells Dev. 2009, 18:1253-1262), and oligodendrocyte precursor cells (Cid C, Alvarez-Cermeno JC, Camafeita E, Salinas M, Alcazar A., FASEB J. 2004;18:409-411) and the like. An "antigen-presenting cell" is a cell that has a function to incorporate an antigen as a foreign substance thereinto, decompose the antigen into peptides, bind the peptides to HLA molecules and express the same on the cell surface to present the antigen to T cells; dendritic cells, macrophages, B cells, monocytes (mononuclear leukocytes) and the like are known to be antigen-presenting cells. The antigen-presenting cells intended for the present invention are preferably dendritic cells, macrophages, monocytes and the like, particularly preferably activated dendritic cells (cells in a state in which an antigen has been incorporated), macrophages and monocytes.

Cell surface HSP90 has also been reported to occur in human tumor cells such as melanoma (Becker B, Multhoff G, Farkas B, et al., Exp Dermatol. 2004, 13:27-32.; Stellas D, Karameris A, Patsavoudi E. Clin Cancer Res. 2007, 13:1831-1838), fibrosarcoma (Eustace BK, Sakurai T, Stewart JK, et al., Nat Cell Biol. 2004; 6:507-514), and neuroblastoma (Cid C, Regidor I, Poveda PD, Alcazar A., Cell Stress Chaperones. 2009 ; 14:321-327).

Therefore, in the present invention, cells expressing cell surface HSP90 include these cells. Preferably, the cells expressing cell surface HSP90 are antigen-presenting cells or tumor cells, particularly preferably activated dendritic cells and macrophages, as well as human tumor cells.

Cell surface HSP90 is present in the vicinity of the cell membrane at least in a manner that enables a reaction with an extracellular anti-HSP90 antibody.

### Anti-HSP90 antibody

The monoclonal antibody of the present invention binds to an epitope comprising the amino acid sequence of SEQ ID NO:1 or 2 shown below, and specifically recognizes HSP90 on the cell surface. The monoclonal antibody of the present invention specifically recognizes either one of HSP90α, and HSP90β. Preferably, the monoclonal antibody of the present invention specifically recognizes both HSP90α and HSP90β. VX₁X₂EX₃PPLEGDX₄ (wherein each of X₁ to X₄, which may be identical to or different from each other, represents an arbitrary amino acid) (SEQ ID NO:1)
HX₅IX₆ETLRQKAE (wherein each of X₅ to X₆, which may be identical to or different from each other, represents an arbitrary amino acid) (SEQ ID NO:2)

In SEQ ID NO:1, each of X₁ to X₄, which may be identical to or different from each other, represents an arbitrary amino acid, wherein it is preferable that X₂ be E or D and X₄ be E or D. In SEQ ID NO:2, each of X₅ to X₆, which may be identical to or different from each other, represents an arbitrary amino acid, wherein it is preferable that X₆ be I or V.

The epitope more preferably comprises:
(1) the amino acid sequence of SEQ ID NO:1 wherein X₁ is T, X₂ is E, X₃ is M, and X₄ is D [i.e., VTEEMPPLEGDD (SEQ ID NO:3)],
(2) the amino acid sequence of SEQ ID NO:1 wherein X₁ is P, X₂ is D, X₃ is I, and X₄ is E [i.e., VPDEIPPLEGDE (SEQ ID NO:4)],
(3) the amino acid sequence of SEQ ID NO:2 wherein X₅ is S, and X₆ is I [i.e., HSIIETLRQKAE (SEQ ID NO:5)], or
(4) the amino acid sequence of SEQ ID NO:2 wherein X₅ is P, and X₆ is V [i.e., HPIVETLRQKAE (SEQ ID NO:6)].

The epitope particularly preferably comprises the amino acid sequence of SEQ ID NO:3 or the amino acid sequence of SEQ ID NO:4.

The amino acid sequence of SEQ ID NO:3 corresponds to the region of positions 712-723 of human HSP90α (NP_005339) or the region of positions 713-724 of mouse HSP90α (NP_034610). The amino acid sequence of SEQ ID NO:4 corresponds to the region of positions 704-715 of human HSP90β (NP_031381) or the region of positions 704-715 of mouse HSP90β (NP_032328). The amino acid sequence of SEQ ID NO:5 corresponds to the region of positions 640-651 of human HSP90α or the region of positions 641-652 of mouse HSP90α. The amino acid sequence of SEQ ID NO:6 corresponds to the region of positions 632-643 of human HSP90β or the region of positions 632-643 of mouse HSP90β.

The antibody of the present invention is not particularly limited, as far as it binds to the above-described epitope and is capable of recognizing HSP90 on the cell surface; such antibodies include, but are not limited to, naturally occurring antibodies; chimeric antibodies, humanized antibodies and single-chain antibodies which can be produced using genetic engineering technology; human antibodies which can be produced using human antibody-producing transgenic animals and the like; antibody fragments prepared using a Fab expression library; and binding fragments thereof. The antibody of the present invention may be, for example, an antibody that recognizes cell surface HSP90, comprising at least one of heavy-chain CDR1 (the amino acid sequence shown by the positions 66 to 70 of SEQ ID NO:10), CDR2 (the amino acid sequence shown by the positions 85 to 101 of SEQ ID NO:10), CDR3 (the amino acid sequence shown by the positions 134 to 141 of SEQ ID NO:10), light-chain CDR1 (the amino acid sequence shown by the positions 43 to 58 of SEQ ID NO:12), CDR2 (the amino acid sequence shown by the positions 74 to 80 of SEQ ID NO:12), and CDR3 (the amino acid sequence shown by the positions 113 to 121 of SEQ ID NO:12), or may be an antibody that recognizes cell surface HSP90, comprising a heavy-chain variable region that comprises the amino acid sequence shown by the positions 36 to 185 of SEQ ID NO:10 and/or a light-chain variable region that comprises the amino acid sequence shown by the positions 20 to 177 of SEQ ID NO:12. For example, a humanized antibody generated by grafting a CDR sequence derived from a non-human mammal to a human framework (FR) sequence, and a human-type antibody in which a variable region sequence derived from a non-human mammal is combined with a human constant region sequence can be used. Methods of generating these antibodies are publicly known; for example, humanized antibodies are described in US Patent Nos. 5225539, 5585089, 5693761, 5693762, and 6180370 and the like. A binding fragment means a partial region of the aforementioned antibody; specific examples include F(ab')₂, Fab', Fab, Fv (variable fragment of antibody), sFv, dsFv (disulphide stabilized Fv), dAb (single domain antibody) and the like (Exp. Opin. Ther. Patents, Vol.6, No.5, pp.441-456, 1996). All these can be used like the monoclonal antibody of the present invention. Monoclonal antibodies are mainly described below; however, when monoclonal antibodies are mentioned herein, the above-described antibodies and fragments thereof are included therein. The above-described antibodies and fragments thereof may be chemically modified with, for example, polyethylene glycol (PEG) and the like.

The monoclonal antibody of the present invention may have one to several amino acids substituted, deleted and/or inserted in the amino acid sequence thereof, as far as it is capable of binding to the above-described epitope, and recognizing HSP90 on the cell surface.

The class of antibody is not particularly limited; antibodies of any isotypes such as IgG, IgM, IgA, IgD and IgE are encompassed. In view of the ease of purification and the like, the class is preferably IgG, more preferably IgG2a.

As the immunogen used to produce the monoclonal antibody of the present invention, a polypeptide consisting of the full-length HSP90 protein, or a portion thereof comprising an epitope, can be used. Specifically, the immunogen is a polypeptide comprising a region consisting of SEQ ID NO:1 or 2, more preferably one amino acid sequence selected from the group consisting of SEQ ID NOs:3 to 6.

For the purpose of crosslinking the above-described polypeptide to a carrier, utilizing the same for purification or the like, one or several amino acids may be added. The number of amino acids to be added is not particularly limited. However, taking into account the specificity of the antibody produced, the number is preferably 1 to 10, more preferably 1 to 5, still more preferably 1 to 2, and most preferably 1.

The position of the amino acid added may be the N-terminus or C-terminus of the polypeptide, and is preferably the N-terminus.

Furthermore, as far as the immunogenicity is maintained, the above-described polypeptide as an immunogen may have one to several amino acids substituted, deleted and/or inserted in the amino acid sequence thereof. The number of amino acids to be substituted, deleted and/or inserted is not particularly limited, and is preferably 1 to 20, more preferably 1 to 10, still more preferably 1 to 5, most preferably 1 to 2.

A polypeptide as an immunogen can be obtained by, for example, a method wherein the desired polypeptide is isolated and purified from an antigen-producing tissue or cells of a mammal (e.g., a human, monkey, rat or mouse) using a known method or a modified method based thereon; a method wherein the desired polypeptide is chemically synthesized by a known peptide synthesis method using a peptide synthesizer or the like; a method wherein a transformant harboring a DNA that encodes the desired polypeptide as an immunogen is cultured; or a method wherein the desired polypeptide is biochemically synthesized using a cell-free transcription/translation system with a nucleic acid that encodes the polypeptide as an immunogen as a template, or the like.

When an immunogen is to be prepared from a mammalian tissue or cells, the immunogen can be isolated and purified by homogenizing the tissue or cells and then extracting the homogenate with acid, alcohol or the like, and subjecting the extract to a known protein separation technique (e.g., salting-out, dialysis, gel filtration, chromatographies such as reversed-phase chromatography, ion exchange chromatography, or affinity chromatography, or the like). The peptide as an antigen obtained may be used as an immunogen as it is, or may be subjected to limited degradation using a peptidase or the like to yield a partial peptide, which may be used as an immunogen.

When a polypeptide as an immunogen is to be chemically synthesized, the synthetic peptide used is, for example, a polypeptide having the same structure as the above-described polypeptide as an immunogen purified from nature, specifically a polypeptide comprising the same amino acid sequence as the amino acid sequence of SEQ ID NO:1 or 2, or one amino acid sequence selected from the group consisting of SEQ ID NOs:3 to 6 in the natural polypeptide as an immunogen, which is the epitope for the monoclonal antibody of the present invention.

When a polypeptide as an immunogen is to be produced using a transformant harboring a DNA, the DNA can be prepared according to a publicly known method of cloning [e.g., method described in Molecular Cloning, 2nd ed.; J. Sambrook et al., Cold Spring Harbor Lab. Press (1989) or the like].

When a cell-free transcription/translation system is to be utilized, a method can be used wherein an mRNA is synthesized using an expression vector having an inserted DNA that encodes a polypeptide as an immunogen which is prepared according to a publicly known method of cloning as described above (for example, an expression vector in which the DNA is placed under the control of T7 promoter, SP6 promoter or the like) as a template, and using a transcription reaction solution which contains an RNA polymerase that is suitable for the promoter and substrate (NTPs), after which a translation reaction is carried out using a publicly known cell-free translation system (e.g., Escherichia coli, rabbit reticulocytes, wheat germ extract or the like) using the mRNA as a template.

Although a polypeptide as an immunogen in an insolubilized form can be used directly as an immunogen as far as it possesses the immunogenicity, the polypeptide as an immunogen can be used for immunization as a complex bound or adsorbed to an appropriate carrier as required. Carriers that can be used include natural or synthetic polymers. Usable natural polymers include serum albumins of mammals (e.g., bovine, rabbit, human, etc.), cycloglobulins of mammals (e.g., bovine, rabbit, etc.), ovalbumin (e.g., of chicken), hemoglobins of mammals (e.g., bovine, rabbit, human, sheep, etc.), keyhole limpet hemocyanin (KLH) and the like. Synthetic polymers include, for example, various latexes of polymers or copolymers such as polyamino acids, polystyrenes, polyacrylics, polyvinyls, or polypropylenes, or the like.

The monoclonal antibody of the present invention can be prepared by a method usually used in the art using the immunogen described above.

### (Preparation of monoclonal antibody)

Specifically, a monoclonal antibody can be produced as described below. A polypeptide as an immunogen (described above) is transplanted or injected once to several times to an animal such as a mouse, rat or hamster subcutaneously, intramuscularly, intravenously, into a footpad or intraperitoneally, whereby the animal is subjected to immune sensitization. Usually, one to four immunizations are performed at intervals of about 1 to 14 days after first immunization; about 1 to 5 days after final immunization, antibody-producing cells are obtained from the immune-sensitized mammal.

A monoclonal antibody is produced by preparing hybridomas (fused cells) from antibody-producing cells which are obtained from an immune-sensitized animal as described above, and a myeloma cell which does not have an ability to produce an antibody by itself, cloning the hybridomas, and selecting a clone that produces a monoclonal antibody that exhibits specific affinity for the immunogen used to immunize the mammal.

Preparation of a hybridoma (fused cell) that secretes a monoclonal antibody can be performed according to the method of Kohler and Milstein et al. (Nature, Vol.256, pp.495-497, 1975) or a modified method based thereon. Specifically, the hybridoma is prepared by cell-fusion of antibody-producing cells contained in a spleen, lymph node, bone marrow, tonsil or the like, preferably in a spleen, obtained from a mammal immune-sensitized as described above, and a myeloma cell which does not have an ability to produce an antibody by itself, preferably derived from a mammal such as a mouse, rat, guinea pig, hamster, rabbit or human, more preferably from a mouse, rat or human.

For example, the mouse-derived myeloma P3/X63-AG8.653 (653; ATCC No.CRL1580), P3./NSI/1-Ag4-1 (NS-1), P3/X63-Ag8.U1 (P3U1), SP2/0-Ag14 (Sp2/0, Sp2), PAI, F0 or BW5147, the rat-derived myeloma 210RCY3-Ag.2.3., or the human-derived myeloma U-266AR1, GM1500-6TG-A1-2, UC729-6, CEM-AGR, D1R11 or CEM-T15 can be used as a myeloma cell for the cell fusion.

Screening for a hybridoma clone that produces a monoclonal antibody can be performed by culturing a hybridoma in, for example, a microtiter plate, and measuring the reactivity of the culture supernatant in a well in which proliferation is observed to the immunogen used in the immune sensitization, by, for example, an enzyme immunoassay such as RIA or ELISA.

Production of a monoclonal antibody from a hybridoma can be achieved by culturing the hybridoma in vitro, or in vivo in the ascites fluid or the like of a mouse, rat, guinea pig, hamster, rabbit or the like, preferably a mouse or rat, more preferably a mouse, and isolating the antibody from the resulting culture supernatant or the ascites fluid of the mammal.

In vitro cultivation can be performed by using a known nutrient medium or any nutrient medium derived or prepared from a known basal medium, which is used to proliferate, maintain, and preserve a hybridoma, and to produce a monoclonal antibody in the culture supernatant, according to various conditions such as the characteristics of the cell to be cultured, the object of the study or research, the method of cultivation and the like.

Furthermore, since the monoclonal antibody of the present invention needs to possess an epitope-binding property and the capability to recognize cell surface HSP90, in addition to affinity for the immunogen, the antibody produced by the hybridoma obtained is checked for the presence or absence of these features. Specifically, these features can be evaluated by epitope mapping of the monoclonal antibody produced by each hybridoma. The epitope mapping can be achieved by the peptide array method, the mass spectrometric method, structural determination by NMR or the like. The peptide array method is a method in which the epitope is determined by binding the antibody to a peptide array in which the amino acid sequences from the immunogen being excised while shifting the window are arranged, and identifying which sequence fragment is recognized. The mass spectrometric method is a method in which the masked epitope region is determined by mass spectrometry after the surface hydroxyl group of the antigen-antibody complex is substituted with heavy water. In case of an antigen that does not permit the use of either technique, the binding portion is determined by a structural analysis by NMR.

The method for evaluating the capability to recognize cell surface HSP90 can be performed in accordance with the method for detecting a cell expressing HSP90 on the cell surface as described below.

Thus, a monoclonal antibody whose epitope is SEQ ID NO:1 or 2, preferably one selected from the group consisting of SEQ ID NOs:3 to 6 and that recognizes cell surface HSP90 is obtained, and a hybridoma that produces the monoclonal antibody is obtained, which in turn can be used as the monoclonal antibody of the present invention and the hybridoma of the present invention, respectively.

The monoclonal antibody is preferably isolated and/or purified. Isolation/purification of the monoclonal antibody is performed by a method for separating and purifying immunoglobulin [e.g., salting-out, alcohol precipitation, isoelectric precipitation, electrophoresis, adsorption and desorption with an ion exchanger (e.g., DEAE), ultracentrifugation, gel filtration, a method of specific purification in which only the antibody is collected using a solid phase having the antigen being bound or an activated adsorbent such as protein A or protein G (which varies depending on the class of monoclonal antibody), and the bond is dissociated to give the antibody].

The monoclonal antibody of the present invention may be labeled with a detectable marker. The labeling may be fluorescence labeling, labeling with a low molecular weight compound, labeling with a peptide or the like.

### (Hybridoma)

The present invention provides a hybridoma that produces a monoclonal antibody that recognizes cell surface HSP90. The hybridoma is prepared as described specifically in the foregoing section (Preparation of monoclonal antibody). Specific examples include hybridomas that were newly isolated by the present inventors and have been deposited at the International Patent Organism Depositary, National Institute of Advanced Industrial Science and Technology (independent administrative corporation) (Tsukuba Central 6, 1-1-1, Higashi, Tsukuba, Ibaraki, Japan) under the accession numbers FERM BP-11222 and FERM BP-11243 (deposit dates: January 13, 2010 and March 11, 2010, respectively), and the like.

Accordingly, the antibody of the present invention can be a monoclonal antibody produced by the above-described hybridoma whose accession number is FERM BP-11222 or FERM BP-11243. The monoclonal antibody produced by the above-described hybridoma whose accession number is FERM BP-11222 comprises a heavy-chain variable region (VH) comprising the amino acid sequence shown by the positions 36 to 185 of SEQ ID NO:10 and/or a light-chain variable region (VL) comprising the amino acid sequence shown by the positions 20 to 177 of SEQ ID NO:12. A chimeric (human-type) antibody whose regions other than the variable region are of human origin can be generated using either of these sequences. Furthermore, the above-described monoclonal antibody comprises the amino acid sequences shown by the positions 66 to 70, positions 85 to 101, and positions 134 to 141 of SEQ ID NO:10, the positions 43 to 58, positions 74 to 80, and positions 113 to 121 of SEQ ID NO:12 as heavy-chain CDR1, CDR2, CDR3, light-chain CDR1, CDR2, and CDR3, respectively. Utilizing any one of these sequences, a humanized antibody whose regions other than CDR are of human origin can be generated. A monoclonal antibody that binds to the same epitope as the epitope to which the antibody binds, and that recognizes cell surface HSP90 can also be suitably used as the antibody of the present invention.

Furthermore, the antibody of the present invention can also be produced by genetic engineering on the basis of information on the base sequences of the heavy-chain and light-chain thereof. For example, the antibody can be obtained from a culture obtained by introducing an expression vector comprising the sequence of SEQ ID NO:9 and/or 11 into a host, and culturing the host. Here, SEQ ID NO:9 and/or 11 may be incorporated into the same expression vector, or may be incorporated into separate expression vectors.

Cells that express the antibody of the present invention or a functional fragment thereof can be prepared using an expression vector constructed to express the antibody of the present invention. Specifically, DNA coding for the antibody of interest is incorporated into an expression vector. In this step, DNA is incorporated into an expression vector so that that it is expressed under control of an expression control region, for example, an enhancer and a promoter. Next, a host cell is transformed with the expression vector to express the antibody. In this process, a suitable combination of a host and an expression vector may be used.

Examples of the vector include M13 vectors, pUC vectors, pBR322, pBluescript, pCR-Script. For the purpose of subcloning and excising cDNA, for example, pGEM-T, pDIRECT and pT7 may also be used besides the above-mentioned vectors.

When a vector is to be used for the purpose of producing an antibody, an expression vector is especially useful. For example, when E. coli such as JM109, DH5α, HB101 or XL1-Blue is used as a host, it is indispensable that the expression vector has a promoter that can efficiently drive the expression in E. coli , for example, lacZ promoter (Ward et al., Nature (1989) 341, 544-546; FASEB J. (1992) 6, 2422-2427, hereby incorporated by reference in its entirety), araB promoter (Better et al., Science (1988) 240, 1041-1043, hereby incorporated by reference in its entirety) or T7 promoter. Such vectors include pGEX-5X-1 (Pharmacia), "QIA express system" (QIAGEN), pEGFP, and pET (in this case, the host is preferably BL21 in which T7 RNA polymerase is expressed) besides the above-mentioned vectors.

The vector may comprise a signal sequence for polypeptide secretion. As the signal sequence for polypeptide secretion, for example, pelB signal sequence (Lei, S. P. et al., J. Bacteriol. (1987) 169, 4397, hereby incorporated by reference in its entirety) may be used in the case of production in periplasm of E. coli. The introduction of the vector into a host cell may be effected, for example, using a calcium chloride method or an electroporation method.

In addition, mammal-derived expression vectors (e.g., pcDNA3 (Invitrogen), pEGF-BOS (Nucleic Acids, Res., 1990, 18(17), p. 5322, hereby incorporated by reference in its entirety), pEF, pCDM8); insect cell-derived expression vectors (e.g., "Bac-to-BAC baculovirus expression system" (GIBCO BRL), pBacPAK8); plant-derived expression vectors (e.g., pMH1, pMH2); animal virus-derived expression vectors (e.g., pHSV, pMV, pAdexLcw), retrovirus-derived expression vectors (e.g., pZIPneo), yeast-derived expression vectors (e.g., "Pichia Expression Kit" (Invitrogen), pNV11, SP-Q01), Bacillus subtilis-derived expression vectors (e.g., pPL608, pKTH50), and the like may be used.

For expression in animal cells such as CHO cells, COS cells or NIH3T3 cells, it is indispensable that the vector has a promoter necessary for expression in the cells, for example, SV40 promoter (Mulligan et al., Nature (1979) 277, 108, hereby incorporated by reference in its entirety), MMTV-LTR promoter, EF1α promoter (Mizushima et al., Nucleic Acids Res. (1990) 18, 5322, hereby incorporated by reference in its entirety), CAG promoter (Gene (1991) 108, 193, hereby incorporated by reference in its entirety), CMV promoter. Preferably, the vector has a gene for selection of the transformed cells (e.g., drug resistance gene that enables discrimination by drug (e.g., neomycin, G418)). The vectors having such characteristics include, for example, pMAM, pDR2, pBK-RSV, pBK-CMV, pOPRSV, pOP13.

Further, for the purpose of stable gene expression and amplification of the copy number of a gene in cells, a method may be used in which a vector having a DHFR gene (e.g., pCHOI) is introduced into CHO cells, which are deficient in the nucleic acid synthetic pathway, to complement the deficiency and is amplified with methotrexate (MTX). For the purpose of transient expression of a gene, a method may be used in which COS cells which have an SV40 T antigen-expressing gene on the chromosome are transformed with a vector having SV40 replication origin (e.g., pcD). A replication origin derived from polyoma virus, adenovirus, bovine papilloma virus (BPV), etc. may be used. Further, for amplifying the copy number of a gene in a host cell system, the expression vector may contain, as a selection marker, aminoglycoside transferase (APH) gene, thymidine kinase (TK) gene, E. coli xanthine-guanine phosphoribosyl transferase (Ecogpt) gene, dihydrofolate reductase (dhfr) gene or the like.

### Method of detecting cell surface HSP90

The present invention provides a method of detecting a cell expressing HSP90 on the cell surface. The method comprises (i) the step of reacting the antibody of the present invention (in particular, monoclonal antibody) that binds to an epitope consisting of SEQ ID NO:1 or 2, preferably one amino acid sequence selected from the group consisting of SEQ IDs NO:3 to 6, and that recognizes cell surface HSP90, or the monoclonal antibody of the present invention produced by the hybridoma of the present invention whose accession number is FERM BP-11222 or FERM BP-11243, with a cell not subjected to permeabilization, and (ii) the step of assessing the presence or absence of the formation of a complex of the antibody and cell surface HSP90.

A "permeabilization" refers to a treatment by which holes are drilled in the cell membrane such that large molecules (e.g., antibodies) are able to enter into cells, and is carried out using a method well known in the art (e.g., a method in which a surfactant is used, a method in which an organic solvent is used, or the like).

Because the cell for which the expression of HSP90 is to be detected in the present invention has not been subjected to such membrane permeabilization, an antibody does not enter into the cell and hence does not react with HSP90 in the cell. Accordingly, the method of the present invention has been made on the basis of the finding that the monoclonal antibody of the present invention is capable of recognizing cell surface HSP90, and makes it possible to detect a cell expressing cell surface HSP90. While HSP90 expressed in cytoplasm occurs universally in mammalian cells, HSP90 expressed on the cell surface occurs only in limited kinds of cells such as antigen-presenting cells and tumor cells, as stated above. Therefore, the detection method of the present invention makes it possible to specifically detect these cells. Further, since the detection method of the present invention does not require membrane permeabilization of cells, it can be used for living cells.

Hereinafter, the respective steps are described.

### (i) The step of reacting the monoclonal antibody of the present invention with a cell not subjected to permeabilization

The monoclonal antibody used in this step is the above-described monoclonal antibody of the present invention, specifically a monoclonal antibody that binds to an epitope consisting of SEQ ID NO:1 or 2, preferably one amino acid sequence selected from the group consisting of SEQ IDs NO:3 to 6, and that is capable of recognizing cell surface HSP90.

The cell to be reacted with the monoclonal antibody of the present invention can be any animal cell for which it is desired to examine whether or not HSP90 is expressed on the cell surface.

The reaction conditions for reacting a monoclonal antibody with a cell (e.g., concentrations of the antibody and the cell, reaction temperature, reaction time, composition of the buffer used, and the like) are known to those skilled in the art; for example, the reaction is carried out using an excess amount of the antibody for the cell, at 0°C to 20°C, preferably at about 4°C, for 0.5 to 1 hour, in a buffer such as an isotonic phosphate buffer.

### (ii) The step of assessing the presence or absence of the formation of a complex of the antibody and HSP90 expressed on the cell membrane

This step can be carried out in the same manner as a method of detecting a complex formed in a usual antigen-antibody reaction. For example, known methods such as RIA, surface plasmon resonance, protein chips, and flow cytometry can be used. In these methods, the monoclonal antibody of the present invention may be labeled in advance for the sake of its detection, or a labeled secondary antibody capable of recognizing the antibody of the present invention may be used. Labeling methods include fluorescence labeling, labeling with a low molecular weight compound, labeling with a peptide, and the like. All these methods are conventionally carried out in the art; those skilled in the art are able to choose and perform an appropriate method taking the purpose thereof into account. For example, since a cell not subjected to membrane permeabilization, preferably a living cell, is used in the present invention, it is suitable to use a method in which a flow cytometer is used, that is, flow cytometry (FACS).

As stated above, the monoclonal antibody of the present invention is capable of recognizing HSP90 on the cell surface. Therefore, the fact that the formation of a complex of the antibody and HSP90 not subjected to membrane permeabilization is observed means that HSP90 is present on the cell surface.

Cells for which cell surface HSP90 is detected include antigen-presenting cells (described above) and tumor cells, specifically antigen-presenting cells such as activated dendritic cells and macrophages and human-derived tumor cells. Therefore, the method of the present invention for detecting/identifying a cell expressing HSP90 on the cell surface is suitable as a method of detecting an antigen-presenting cell such as an activated dendritic cell or macrophage, or a method of detecting/identifying a human-derived tumor cell.

The anti-HSP90 antibodies of the present invention other than monoclonal antibodies can also be used in the above-described detection method like the monoclonal antibodies of the present invention.

### Vaccine

The present invention provides a vaccine comprising a complex of a monoclonal antibody that recognizes HSP90 on the cell surface and a target antigen (hereinafter also simply referred to as the "antigen-antibody complex" of the present invention for convenience). Preferably, the monoclonal antibody is the monoclonal antibody of the present invention (described above). More preferably, the monoclonal antibody recognizes the amino acid sequence of SEQ ID NO:3 (VTEEMPPLEGDD) as an epitope. Specifically, the monoclonal antibody is, for example, an antigen-binding site that comprises the light-chain and/or heavy-chain variable region or any one of the CDRs thereof of the monoclonal antibody produced by the hybridoma whose accession number is FERM BP-11222 (6H8D2; also referred to as 6H8 for convenience), or an antibody containing the same. Here, an antigen-binding site refers to a site that binds to an antigen in an antibody; for example, a Fab (Fragment, antigen binding) region which comprises a light-chain constant region and a light-chain variable region can be used as a polypeptide that comprises an antigen-binding site. Here, a light-chain variable region refers to a region on the N-terminus side of the light-chain Fab region comprising a sequence required for recognizing an antigen.

As described in Examples below, the monoclonal antibody, upon binding to HSP90 on an antigen-presenting cell, forms an antibody-HSP90 complex, and the complex is efficiently internalized (incorporated) into the cell. Therefore, a complex of a monoclonal antibody that recognizes HSP90 on the cell surface (preferably the monoclonal antibody of the present invention) and a target antigen binds to HSP90 on the cell surface and is then incorporated into the cell.

The target antigen in the complex is internalized as the complex is incorporated in the cell. Subsequently, the antigen is delivered to the MHC class I antigen presentation pathway, where cross-presentation occurs to activate CD8⁺ T cells. By this action mechanism, the complex functions as a vaccine. Therefore, the vaccine of the present invention can be used in a method of inducing immunity to a target antigen.

According to the present invention, there is provided a method of treating or preventing a disease, comprising the step of administering an effective amount of the vaccine of the present invention to a subject. A target antigen can be selected from any publicly known antigens according to the disease to be prevented or treated.

For example, a target antigen is not particularly limited as far as it is an antigen that is expressed on an abnormal cell or pathogen, and disappearance or decrease in the amount of the abnormal cell or pathogen is expected as a result of an immunological action targeting the antigen. Examples of the target antigen include tumor antigens and pathogen antigens.

The tumor antigen may be an antigen of a solid tumor (including epithelial and nonepithelial tumors), or a tumor in a hematopoietic tissue. Examples of the solid tumor antigen include, but are not limited to, MART-1/Melan-A, Mage-1, Mage-3, gp100, tyrosinase, tyrosinase-related protein 2 (trp2), CEA, PSA, CA-125, erb-2, Muc-1, Muc-2, TAG-72, AES, FBP, C-lectin, NY-ESO-1, galectin-4/NY-CO-27, Pec60, HER-2/erbB-2/neu, telomerase, G250, Hsp105, point mutation ras oncogene, point mutation p53 oncogene and carcinoembryonic antigen (e.g., see JP-A-2005-139118, JP-A-2004-147649, JP-A-2002-112780, JP-A-2004-222726). Examples of the antigen of tumor in a hematopoietic tissue (e.g., leukemia) include, but are not limited to, proteinase 3, WT-1, hTERT, PRAME, PML/RAR-a, DEK/CAN, cyclophilin B, TEL-MAL1, BCR-ABL, OFA-iLRP, Survivin, idiotype, Sperm protein 17, SPAN-Xb, CT-27 and MUC1.

The pathogen antigen may be a pathogenic virus antigen, a pathogenic microorganism antigen, or a pathogenic protozoan antigen. Examples of the pathogenic virus antigen include, but are not limited to, antigens of viruses such as human immunodeficiency virus (HIV), hepatitis viruses (e.g., hepatitis A, B, C, D and E viruses), influenza viruses, herpes simplex virus, West Nile fever virus, human papillomavirus, equine encephalitis virus, human T-cell leukemia virus (e.g., HTLV-I) and the like. Specific examples include GP-120, p17, GP-160 (which are of HIV), NP, HA (which are of influenza virus), HBs Ag, HBV envelope protein, core protein, polymerase protein, NS3, NS5 (which are of hepatitis virus), HSVdD (of herpes simplev virus), EBNA1, 2, 3A, 3B and 3C, LMP1 and 2, BZLF1, BMLF1, BMRF1, BHRF1 (which are of EB virus), Tax (of HTLV-I), SARS-CoV spike protein (of SARS virus), CMV pp5, IE-1 (which are of CMV), E6, E7 proteins (which are of HPV) (e.g., see JP-A-2004-222726). Examples of the pathogenic microorganism antigen include antigens expressed on pathogenic bacteria (e.g., Chlamydia, Mycobacterium, Legionella) and pathogenic yeasts (e.g., Aspergillus, Candida). Examples of the pathogenic protozoan antigen include antigens expressed on malaria and schistosome.

In the antigen-antibody complex of the present invention, the binding between the monoclonal antibody that recognizes cell surface HSP90 and a target antigen may be a covalent bond or a non-covalent bond, and may be direct or indirect. To bind the two components, various methods publicly known in the art can be used.

Examples of covalent bonds include, but are not limited to, one formed when a maleimidated antigen is reacted with an antibody to form a complex.

Examples of non-covalent bonds include, but are not limited to, one formed when an antibody and an antigen are indirectly coupled utilizing the binding between biotin and streptavidin by binding biotin to one of the antibody or the antigen, and binding streptavidin to the other. To achieve an indirect binding of an antibody and an antigen, a nano-carrier such as a liposome can be utilized. In this case, an antigen-antibody complex can be prepared by binding the antigen to the surface of a nano-carrier or encapsulating the antigen in the nano-carrier to bind the antibody on the surface of the nano-carrier with the antigen-binding site facing the outside. The composition of the nano-carrier is not particularly limited, and can be selected from those publicly known in the art. The binding between the antibody or antigen and the nano-carrier can also be achieved using a method publicly known in the art.

The antigen-antibody complex of the present invention can be administered to a patient in an amount effective in functioning as a vaccine. Those skilled in the art are able to determine the effective amount without the need for undue experimentation, for example, on the basis of an exo vivo test using a cell line, a test using a laboratory animal, or the like.

The vaccine of the present invention is useful for neoplastic diseases or infectious diseases.

Examples of the neoplastic disease include solid tumors (e.g., epithelial tumor, non-epithelial tumor), and tumors in hematopoietic tissues. More specifically, examples of the solid tumor include gastrointestinal cancer (e.g., gastric cancer, colon cancer, colorectal cancer, rectal cancer), lung cancer (e.g., small cell cancer, non-small cell cancer), pancreatic cancer, kidney cancer, liver cancer, thymus, spleen, thyroid cancer, adrenal gland, prostate cancer, urinary bladder cancer, ovarian cancer, uterus cancer (e.g., endometrial carcinoma, cervical cancer), bone cancer, skin cancer, sarcoma (e.g., Kaposi's sarcoma), melanoma, blastoma (e.g., neuroblastoma), adenocarcinoma, planocellular carcinoma, non-planocellular carcinoma, brain tumor, as well as recurrence and metastasis of these solid tumors. Examples of the tumor in hematopoietic tissues include leukemia (e.g., acute myeloid leukemia (AML), chronic myeloid leukemia (CML), acute lymphocytic leukemia (ALL), chronic lymphocytic leukemia (CLL), adult T cell leukemia (ATL), myelodysplastic syndrome (MDS)), lymphoma (e.g., T lymphoma, B lymphoma, Hodgkin's lymphoma), myeloma (multiple myeloma), as well as recurrence of these tumors. Examples of the infectious disease include infections caused by the aforementioned pathogens.

The vaccine of the present invention may optionally comprise, in addition to the above-described antigen-antibody complex, a pharmaceutically acceptable excipient or additive. Pharmaceutically acceptable excipients and additives include carriers, binders, flavorings, buffering agents, thickening agents, coloring agents, stabilizers, emulsifiers, dispersing agents, suspending agents, antiseptics and the like. Examples of the pharmaceutically acceptable carrier include magnesium carbonate, magnesium stearate, talc, sugar, lactose, pectin, dextrin, starch, gelatin, tragacanth, methylcellulose, sodium carboxymethylcellulose, low-melting wax, cacao butter and the like.

The vaccine of the present invention is administered orally or parenterally. When administered orally, the vaccine can be administered in a dosage form usually used in the art. When administered parenterally, the vaccine can be administered in a dosage form such as a preparation for topical administration (e.g., transdermal preparation), a preparation for rectal administration, an injection, a transnasal preparation or the like.

To enhance the immunological effect thereof, the vaccine of the present invention can be administered along with an adjuvant. Generally, an adjuvant refers to a substance that, when used along with an antigen, plays a role in non-specifically increasing immune responses to the antigen, and enhancing cellular immunity and antibody production against the antigen. Examples of the adjuvant include, but are not limited to, complete Freund adjuvant (CFA), incomplete Freund adjuvant (IFA), sodium hydroxide, aluminum hydroxide, substances derived from bacteria or parasites (dead cells of Propionibacterium acnes and the like) and the like. The antibody of the present invention can be administered as an adjuvant along with the vaccine of the present invention. To "administer along with" means coadministration or combination administration; examples include a case where the vaccine of the present invention and an adjuvant are both present in a single composition, a case where the vaccine of the present invention and an adjuvant are present in separate compositions and administered at almost the same time but at different sites, and a case where the vaccine of the present invention and an adjuvant are present in separate compositions and administered at different times. When administered at different times, either the vaccine of the present invention or an adjuvant may be administered in advance, but it is preferable that the adjuvant be administered in advance. A dosing interval can be set arbitrarily in the range of several seconds, several minutes, several hours, or several days.
An adjuvant is administered in an amount effective in enhancing the immunological effect of the vaccine of the present invention. Those skilled in the art are able to determine an effective amount without the need for undue experimentation, for example, on the basis of a test using a laboratory animal, or the like.

Preparations suitable for oral administration include liquid preparations in which an effective amount of a substance is dissolved in a diluent such as water or saline, capsules, granules, powders or tablets containing an effective amount of a substance as a solid or a granule, suspensions in which an effective amount of a substance is suspended in a suitable dispersing medium, emulsions in which a solution of an effective amount of a substance dissolved therein is dispersed and emulsified in a suitable dispersing medium, and the like.

Preparations suitable for parenteral administration (e.g. intravenous injection, subcutaneous injection, intramuscular injection, local injection etc.) include aqueous or non-aqueous isotonic sterile injection liquid preparations, which may contain antioxidants, buffers, bacteriostatic agents, tonicity agent etc. In addition, the examples include aqueous and non-aqueous sterile suspensions, which may contain suspending agents, solubilizers, thickening agents, stabilizers, antiseptics etc. The preparation can be sealed in a container such as an ampoule or a vial as a unit dose or multiple doses. Alternatively, an active ingredient and a pharmaceutically acceptable carrier can be lyophilized and stored so that they are dissolved or suspended in a suitable sterile vehicle immediately before use.

The anti-HSP90 antibodies of the present invention other than monoclonal antibodies can also be used in the above-described vaccine like the monoclonal antibodies of the present invention.

### Drug

The present invention provides a drug comprising a complex of an anti-HSP90 monoclonal antibody capable of recognizing cell surface HSP90 and a substance possessing a biological activity bound thereto.

A complex of an antibody and a substance possessing a biological activity (hereinafter also referred to as a "substance-antibody complex" for convenience) in the drug of the present invention, like in the vaccine of the present invention, is capable of being internalized into cells. The substance is liberated from the internalized substance-antibody complex by decomposition or through other mechanisms, thereby the biological activity of the substance is exhibited. Therefore, in the drug of the present invention, the substance possessing a biological activity can be efficiently delivered into cells.

For explanations of the antibody, binding between the antibody and the substance, drug formulation, and the method of administration, the corresponding description of the above-described vaccine of the present invention is applicable.

The substance possessing a biological activity is not specifically limited as far as it has a biological activity, and may be a publicly known substance or a novel substance that would be developed in the future. The substance possessing a biological activity may be a compound or a composition. The compounds include low molecular weight compounds, high molecular weight compounds and the like. The low molecular weight compound is a compound having a molecular weight of less than about 1000; examples include organic compounds, derivatives thereof, and inorganic compounds, that can be usually used as pharmaceuticals. It is a compound produced using a method of organic synthesis or the like, or a derivative thereof, a naturally occurring compound, or a derivative thereof, a small nucleic acid molecule such as a promoter, one of various metals, or the like; the low-molecular weight compound desirably refers to an organic compound or a derivative thereof, or a nucleic acid molecule that can be used as a pharmaceutical. A high molecular weight compound is a compound having a molecular weight of about 1000 or more; examples include proteins, polynucleic acids, polysaccharides, combinations thereof and the like. The composition may comprise two or more of the above-described compounds, and may comprise as required an inert carrier that does not adversely affect the biological activity and the like. These substances may be commercially available if they are publicly known products, or may be obtained through the steps of collection, production, purification and the like according to reports or literatures. These may be naturally occurring, prepared using genetic engineering techniques, or obtained by semi-synthetic processes or the like.

In view of the recognition of HSP90 on the cell surface and the internalization into the cell along with the antibody, the substance to be coupled to the antibody is preferably a substance that exhibits a biological activity on a cell expressing HSP90 on the cell surface. For example, in view of the expression of HSP90 on human tumor cells, an anticancer agent can be used as the substance. Anticancer agents include alkylating agents such as cyclophosphamide and triethylene melamine, metabolic antagonists such as mercaptopurine, 5-fluorouracil and methotrexate, anti-tumor antibiotics such as daunomycin and adriamycin, alkaloids, hormones and the like. Furthermore, in view of the exertion of functioning after entering into cells, a nucleic acid molecule can be used as the substance. The nucleic acid molecule may be either DNA or RNA. DNA may comprise a gene so that the gene can be expressed, and examples include plasmids, gene constructs comprising a gene linked to a promoter, and the like. DNAs include antisense DNAs, genes encoding enzyme inhibitors or polypeptides possessing cytotoxicity or apoptosis inducing action, genes encoding inhibitors of transcription factors, and the like. RNAs include siRNAs, antisense RNAs, shRNAs and the like. In particular, siRNAs are suitable for use in the drug of the present invention because they possess the well known function in controlling gene expression.

Using the drug of the present invention, it is possible to treat or prevent a disease or condition (e.g., tumor) that can be treated or prevented with a substance exhibiting a biological activity. Accordingly, the present invention provides a method of treating a disease, comprising administering an effective amount of the drug of the present invention to a subject in need thereof.

The anti-HSP90 antibodies of the present invention other than monoclonal antibodies can also be used in the above-described drug like the monoclonal antibodies of the present invention.

### Adjuvant and use thereof

The present invention provides an adjuvant comprising a polypeptide that comprises an antigen-binding site of a monoclonal antibody that recognizes HSP90 on the cell surface. The monoclonal antibody is preferably the above-described monoclonal antibody of the present invention. More preferably, the monoclonal antibody recognizes the amino acid sequence of SEQ ID NO:3 (VTEEMPPLEGDD) as an epitope. Specifically, the monoclonal antibody is, for example, an antigen-binding site comprising the light-chain and/or heavy-chain variable region or any one of the CDRs thereof of a monoclonal antibody produced by the hybridoma whose accession number is FERM BP-11222 (6H8D2; also referred to as 6H8 for convenience), or an antibody comprising the same. Here, an antigen-binding site refers to a site that binds to an antigen in an antibody; for example, a Fab (Fragment, antigen binding) region which comprises a light-chain constant region and a light-chain variable region can be used as a polypeptide that comprises an antigen-binding site. Here, a light-chain variable region refers to a region on the N-terminus side of a light-chain Fab region comprising the sequence required for recognizing the antigen.

The adjuvant of the present invention is capable of enhancing the antigen presentation mechanism via binding to an Fc receptor, as described in Examples below. FcRγ, which forms a complex with HSP90 on the cell surface, plays a key role in the action mechanism. FcRγ is known to exhibit actions in immunity induction, including enhancement of the phagocytotic potential, production and secretion of cytokines/chemokines, induction of oxidative burst, and induction of antibody dependent cell mediated cytotoxicity (ADCC). Therefore, the adjuvant of the present invention can be used in a method of inducing immunity based on enhancement of such antigen presentation mechanism.

The adjuvant of the present invention can be administered to a subject along with a vaccine that is incorporated into antigen-presenting cells (preferably dendritic cells) via binding to an Fc receptor. Here, the vaccine used comprises, for example, a complex of a target antigen and an antibody (preferably a monoclonal antibody). The antibody is not particularly limited, as far as it has an Fc portion and is capable of binding to an Fc receptor on antigen-presenting cells (preferably dendritic cells). The antibody is preferably IgG which is capable of binding to an Fcγ receptor. Examples of the target antigen include the target antigens listed in the explanation of the vaccine of the present invention given above, and the target antigen is preferably a pathogen antigen or a tumor antigen.
It is also preferable to use the vaccine of the present invention as the vaccine.
In the complex, any site may be selected as the site of binding between the antibody and the target antigen, as far as it does not interfere with the binding between the antibody and an Fc receptor. The binding between the two components may be a covalent bond or a non-covalent bond; the binding reaction can be carried out using various methods publicly known in the art.

The adjuvant of the present invention can be administered along with an anti-tumor antigen antibody. Here, the anti-tumor antigen antibody is exemplified by the antibody that recognizes a tumor antigen as described above with respect to the vaccine of the present invention.
The anti-tumor antigen antibody forms a complex with a tumor antigen, and the complex is incorporated into dendritic cells via an Fc receptor, where an immunoreaction to the tumor antigen is induced. The adjuvant of the present invention is capable of enhancing such immunity induction mechanism.

The adjuvant of the present invention can be administered along with an anti-tumor cell antibody. An anti-tumor cell antibody means an antibody that has effects of binding to a tumor cell membrane surface and killing tumor cells. Such an antibody, when acting on tumor cells, causes cell death (apoptosis). The cell fractions (apoptotic bodies) of tumor cells or the like which have been converted into pieces as a result of the apoptosis are incorporated via an Fc receptor, resulting in the induction of immunoreactions. Since a tumor cell (or a portion thereof) contains many tumor antigens, a tumor cell treated with an anti-tumor cell antibody functions as a polyvalent vaccine to induce immunoreactions to the tumor cell. Anti-tumor cell antibodies include, but are not limited to, anti-NY-ESO-1 antibodies, anti-MAGE-3 antibodies, and anti-survivin antibodies.
The adjuvant of the present invention is capable of promoting such incorporation into dendritic cells.

For explanations of the methods of administration of the adjuvant and vaccine of the present invention to a subject in the above-described method of inducing immunity, the corresponding description of the vaccine of the present invention given above is applicable.

The adjuvant of the present invention can also be used in a method of inducing immunity comprising the step of administering an anticancer agent to a subject. A tumor antigen is released from a cancer cell disrupted due to an attack by the anticancer agent. This tumor antigen binds to an anti-tumor antigen antibody produced by the subject against the antigen; the resulting antigen-antibody complex is incorporated into dendritic cells via an Fc receptor. The adjuvant of the present invention effectively promotes this incorporation.
Examples of the anticancer agent include the anticancer agents listed in the explanation of the drug of the present invention given above.

The adjuvant of the present invention is administered in an effective amount so that a monoclonal antibody that recognizes HSP90 on the cell surface, particularly the antigen-binding site thereof, preferably a monoclonal antibody provided according to the present invention, particularly the antigen-binding site thereof, contained therein enhances the antigen presentation mechanism via an Fc receptor, that is, exerts an adjuvant effect. Those skilled in the art are able to appropriately determine the effective amount without undue experimentation, for example, on the basis of a test using an animal or the like.

The anti-HSP90 antibodies of the present invention other than monoclonal antibodies can also be used in the above-described adjuvant like the monoclonal antibodies of the present invention.

### Examples

The present invention is hereinafter described in more detail with reference to the following examples, which do not limit the scope of the present invention by any means. The reagents, apparatuses and materials used in the present invention are commercially available unless otherwise specified.

### Example 1

Described in this Example is how to generate anti-HSP90 monoclonal antibodies and hybridomas that produce the same.
A BALB/c mouse was twice immunized with 50 µg of human recombinant HSP90α (prepared using an Escherichia coli expression vector supplied by Professor Takayuki Nemoto at the Nagasaki University School of Dentistry; see J. Biol. Chem. 272:26179-26187, 1997). The adjuvant used was 50 µl of TiterMax Gold (TiterMax, Georgia, USA). Three days after final immunization, splenocytes were taken out from the immunized mouse and fused with P3U1 myeloma cells in a 1:1 ratio by means of polyethylene glycol 4000. The fused cells were seeded to a 96-well plate at 5000 cells/well and cultured in an HAT selection culture broth (an RPMI containing 10% FCS, supplemented with 0.1 mM hypoxanthine, 0.016 mM thymidine, and 0.4 nM aminopterin) for 10 days. The antibodies against HSP90α present in the supernatants in wells containing proliferated cells were detected by ELISA. The hybridomas secreting the antibodies against HSP90α were re-seeded at 0.2 cells/well and further cultured using an HT selection culture broth (an RPMI containing 10% FCS, supplemented with 0.1 mM hypoxanthine and 0.016 mM thymidine) for 10 days. For the antibodies in the supernatants of proliferated cells, it was confirmed using ELISA and Western blotting that antibodies against HSP90 were secreted. The results of the Western blot analysis are shown in Fig. 1A. The DC2.4 dendritic cell line extract was developed on SDS-PAGE and then blotted using each anti-HSP90 antibody obtained [2A6E9 (2A6), 6H8D2 (6H8), 5H12B7 (5H12), or 4B6G12 (4B6)]. All the monoclonal antibodies detected HSP90 as a single band with a size corresponding to HSP90 detected using a commercially available monoclonal antibody (lane 5, SPA-840, Stressgen).
A hybridoma that produces the 6H8 antibody and a hybridoma that produces the 5H12 antibody were deposited to the International Patent Organism Depositary, National Institute of Advanced Industrial Science and Technology (independent administrative corporation) (Tsukuba Central 6, 1-1-1, Higashi, Tsukuba, Ibaraki, Japan). They have been given the accession numbers FERM BP-11222 and FERM BP-11243, respectively (deposit dates: January 13, 2010 and March 11, 2010, respectively).

### Example 2

Described in this Example is the fact that the antibody of the present invention recognizes cell surface HSP90 on cells expressing cell surface HSP90.
Using anti-HSP90 antibodies obtained from a plurality of hybridomas generated by the present inventors (2A6, 6H8, 5H12, 4B6), the cell membrane surface of a GM-CSF-dependent bone marrow-derived dendritic cell (GMCSF-BMDC)-like dendritic cell line DC2.4 (supplied by Dr. Ken Rock at the Massachusetts Institute of Technology) was stained as described below.
(1) 1.1 mg of the biotinylation reagent EZ-Link (registered trademark) Sulfo-NHS-Biotin (21217, Takara Bio Inc.) was dissolved in 250 µl of pure water (concentration 10 mM). 2 µl of the solution of the biotinylation reagent was added to 100 µl of an antibody protein (1 mg/ml), and the reaction was allowed to proceed while cooling on ice for 2 hours. After the reaction, the unreacted biotin reagent was removed using Centricon[5k] (Millipore). The reaction product was adjusted to a final concentration of 1 mg/ml and stored at 4°C.
(2) DC2.4 not subjected to membrane permeabilization (1x10⁶ cells/0.1 ml), along with the biotinylated anti-HSP90 antibody (1 µg/ml), was incubated at 4°C for 30 minutes. For negative control, DC2.4 was likewise incubated in an antibody-free PBS.
(3) After the unreacted antibody was removed, DC2.4 (1x10⁶ cells/0:1 ml), along with PE-cy5 streptavidin (eBioscience, 15-4317-82, used at a concentration of 0.5 µg/ml), was incubated at 4°C for 30 minutes.
(4) After the unreacted PE-cy5 streptavidin was removed, the cells were analyzed by FACS (Becton Dickinson).

As shown in the left panel of Fig. 1B, the monoclonal antibodies 6H8D2 (6H8) and 5H12B7 (5H12), among the antibodies examined in the experiments, recognized cell surface HSP90 with high sensitivity. The right panel of Fig. 1B shows a bar graph of mean fluorescence intensity (MFI) for the data in the left panel.

The following antigen-presenting cells were stained using the 6H8 monoclonal antibody as described above; (i) DC2.4, (ii) mouse BMDC (induced with GM-CSF), (iii) mouse BMDC (induced with Flt3L), (iv) human PBMC-derived immature DC (induced from human peripheral blood CD14-positive cells with GM-CSF and IL4), (v) human BMDC-derived mature DC (induced from human peripheral blood CD14-positive cells with GM-CSF and IL4, and matured with TNFα), (vi) mouse peritoneal macrophages [0.5 ml of pristane (Wako Pure Chemical) was intraperitoneally administered to a mouse; 5 days later, recovered as peritoneal lavage] and (vii) human macrophage cell line J774 (ATCC). In Fig. 1C, the grey areas indicate staining data obtained with PE-Cy5 streptavidin alone as negative controls. Fig. 1D shows a bar graph of the MFI of the data in Fig. 1C. As shown in Figs. 1C and D, the 6H8 antibody recognized cell surface HSP90 on cells other than human PBMC-derived immature DC.

### Example 3

Described in this Example is epitope mapping of anti-HSP90 monoclonal antibodies.
Used as anti-HSP90 monoclonal antibodies were the monoclonal antibodies generated in Example 1, i.e., 2A6E9(2A6), 6H8D2(6H8), 5H12B7(5H12) and 4B6G12(4B6), as well as those supplied by Dr. Takayuki Nemoto at Nagasaki University, i.e., K41102, K41107, K41331 and K41009.
Epitope mapping was performed using biotinylated overlapping peptides. The biotinylated overlapping peptides were prepared as described in a publicly known document (J. Immunological Methods 2002;267:27-35). The epitope mapping was performed using a library of 12-amino-acid-long synthetic peptides each having an overlap of four amino acids.
After an avidin-coated plate was blocked with BSA, the above-described biotinylated overlapping peptides (1.25-3.75 mg/ml) were added. The plate was incubated at room temperature for 2 hours, after which it was washed with PBS/Tween 20 to remove the unreacted biotinylated overlapping peptides. Each anti-HSP90 monoclonal antibody (1 µg/ml) was added, and the plate was incubated at room temperature for 2 hours. The plate was washed with PBS/Tween 20 to remove the unreacted antibody, after which anti-mouse IgG-ALP (BD Pharmingen, used at a 2000-fold dilution) was added, and the plate was incubated at room temperature for 1 hour. The plate was washed with PBS/Tween 20 to remove the unreacted anti-mouse IgG-ALP, after which a pNPP solution (KPL, 1 mg/ml) was added, and the plate was incubated at room temperature for 3 hours to cause color development. After the color development, the plate was subjected to measurement of OD405 nm.
In the left panels of Figs. 2A and B, the horizontal axes indicate amino acid numbers of mouse HSP90α; the vertical axes indicate relative values of OD405 obtained by ELISA assay. The region mapped for each anti-HSP90 monoclonal antibody is shown as a range of amino acid numbers on HSP90α. The epitope recognized by 6H8D2 (6H8) was mapped at HSP90α₇₁₃₋₇₂₄ (corresponding to HSP90β₇₀₄₋₇₁₅), and the epitope recognized by 5H12B7 (5H12) was mapped at HSP90α₆₄₁₋₆₅₂ (corresponding to HSP90β₆₃₂₋₆₄₃).

The right panels of Figs. 2A and B show FACS data (histograms) for DC2.4 cells stained with each monoclonal antibody in the same manner as Example 2.

Fig. 2C shows summarized results of epitope mapping. Recognition sites detected or not detected on the surface of DC2.4 cells by corresponding monoclonal antibodies are shown by shaded boxes and grey boxes, respectively.

This Example showed that the epitope recognized by the 6H8 antibody was located at mouse HSP90α₇₁₃₋₇₂₄ (corresponding to HSP90β₇₀₄₋₇₁₅). This region corresponds to human HSP90α₇₁₂₋₇₂₃ (corresponding to HSP90β₇₀₄₋₇₁₅). The homology between the epitopes on HSP90α and β was 66.7%. Although only eight out of the twelve amino acids are identical, the remaining four are classified into the same category. HSP90α₇₁₃₋₇₂₄ is located in the vicinity of the C-terminus; this region does not crystallize in X-ray crystallographic analysis because it is too flexible. Likewise, epitopes for the 2A6E9 and K41009 antibodies, which recognize epitopes located in the vicinity of the C-terminus, are mouse HSP90α₇₂₂₋₇₃₃ and mouse HSP90α₇₀₂₋₇₁₆, respectively, and they recognize regions that overlap mouse HSP90α₇₁₃₋₇₂₄. However, they did not recognize cell surface HSP90. These results suggest the possible presence of an unknown molecule that forms a complex with HSP90 on the cell surface to prevent these epitopes from being recognized by the 2A6E9 or K41009 antibody.

### Example 4

Described in this Example is the fact that the antibody of the present invention binds to both of the two isoforms of cell surface HSP90, i.e., HSP90α and β.
First, the surface of DC2.4 cells was biotinylated. This biotinylation was performed using Pierce EZ-Link Sulfo-NHS-LC-LC (Thermo scientific, 21338) according to the instructions attached to the reagent. The cell membrane was prepared using sub-cellular extraction kit (Calbiochem) and immunoprecipitated using the 2A6E9 antibody. The precipitate was subjected to SDS-PAGE, after which blotting was performed using an antibody that recognizes both HSP90α and β (6B1C4, another antibody obtained in Example 1) or avidin-HRP (Fig. 3A).

As shown in Fig. 3A, HSP90 immunoprecipitated by the 2A6E9 antibody (lane 6B) and biotinylated (lane Avi.) was detected, demonstrating the expression of HSP90 on the surface of DC2.4 cells.

Mammalian HSP90 occurs in two isoforms: HSP90α and β. The following experiment was performed to determine whether not only HSP90α but also HSP90β is recognized by the antibody of the present invention. First, DC2.4 living cells were incubated along with an antibody on ice using 6H8, 2A6 or normal mouse IgG as a negative control. After being thoroughly washed to remove the unreacted antibody, the cells were lysed; the lysate was centrifuged at 10,000xg, and the resulting supernatant was incubated along with Protein G-Sepharose. The bound material was subjected to SDS-PAGE, after which blotting was performed using an anti-HSP90a antibody (SPS-771, Assay designs), an anti-HSP90β antibody (PB-118-P1, Neomarkers), or an antibody that recognizes both HSP90α and β (6B1C4, another antibody obtained in Example 1).

As shown in Fig. 3B, both HSP90α and β were present on the cell surface and recognized by 6H8 (lane 6H in Fig. 3B). Meanwhile, 2A6E9, which recognized and immunoprecipitated HSP90 in the membrane fraction after cell fractionation in Fig. 3A, did not recognize cell surface HSP90α or β on living cells (lane 2A in Fig. 3B). Therefore, the antibody of the present invention is superior to preexisting antibodies in that it can be used to analyze cell surface HSP90 on living cells.

### Example 5

Described in this Example is the fact that the antibody of the present invention is also useful in research on cell surface HSP90.
The above-described Example demonstrated the presence of a mechanism by which the cytoplasmic chaperon HSP90 is expressed on the cell surface of APC. Since HSP90 does not have any signal sequence for targeting itself to the cell membrane or secretion pathway, HSP90 needs to bind to an unknown molecule that comes on the cell surface through a nonconventional secretion pathway to migrate to the cell surface.
Hence, the influences of the HSP90-specific inhibitors radicicol and novobiocin on the expression of cell surface HSP90 were examined. DC2.4 cells were incubated in the presence of OVA (in Fig. 4A, i; 1 mg/ml), radicicola (in Fig. 4A, ii; 25 µM) or novobiocin (in Fig. 4A, iii; 400 µM) with 5% CO₂ at 37°C for 3 hours. Next, the cells were cooled on ice. To detect cell surface HSP90, the cells were stained with the 6H8 antibody and then analyzed by FACS. As shown in Fig. 4A, the HSP90-specific inhibitors radicicol and novobiocin had no or almost no influence on the expression level of surface HSP90. The absence of any influence of the treatment with the HSP90 inhibitors radicicol or novobiocin on the expression of surface HSP90 demonstrates that the mode of binding to HSP90 on the cell surface and the molecule that allows HSP90 to migrate onto the cell surface is resistant to these inhibitors; therefore, HSP90 is possibly directed to migrate to the cell surface by binding to a molecule that is not the normal client protein.

In view of the possibility that cytokines are variation factors for the expression of cell surface HSP90, the influences of various cytokines on the expression of cell surface HSP90 were examined as described below. DC2.4 cells and mouse GM-CSF-dependent BMDC were incubated in the presence (bold lines in Fig. 5A) or in the absence (thin lines in Fig. 5A) of IFNα (500 U/ml), IFNβ (500 U/ml), or IFNγ (50 ng/ml) with 5% CO₂ at 37°C for 24 hours, and cell surface HSP90 stained using the 6H8 antibody was analyzed by FACS. As a result, it was confirmed that the expression of cell surface HSP90 was increased with the addition of IFN α, β, and γ to the culture broth (Fig. 5A). The grey areas show the results of staining with PE-Cy5 alone as negative controls.
Fig. 5B shows the results of treatments with different concentrations of IFN α, β, and γ. The solid bars indicate the MFI for cells treated with the cytokines in Fig. 5A; the grey bars indicate the MFI for cells incubated in the presence of a low concentration of IFNα (100 U/ml), IFNβ (100 U/ml), or IFNγ (10 ng/ml). The open bars indicate the results for the MFI incubated in the absence of the cytokines as negative controls.
Fig. 5C shows the results obtained by treating BMDC with various cytokines in the same manner as the above-described experiment. The solid bars indicate the MFI for BMDC incubated in the presence of high concentrations of IFNs (as in Fig. 5B) or other cytokines (100 ng/ml); the grey bars indicate the MFI for BMDC incubated in the presence of low concentrations of IFNs (as in Fig. 5B) or other cytokines (10 ng/ml). The open bars indicate the MFI for BMDC incubated in the absence of the cytokines as negative controls. With treatments with IL-3, 4, 5, 6, 10, 12, 15, 18, and 23, no significant difference from the negative control was noted.

### Example 6

Described in this Example is the internalization of cell surface HSP90 by the antibody of the present invention.
The behavior of the 6H8-HSP90 complex after being recognized by the 6H8 antibody was examined as described below. First, DC2.4 cells were incubated in the presence of biotinylated 6H8 (1 µg/ml) on ice for 30 minutes and washed. Next, the cells were incubated at 37°C with 5% CO₂ for 0, 10, 20, 30, or 60 minutes. After cooling on ice, the cells were stained with PE-Cy5-streptavidin and analyzed by FACS. The results are shown in the left panel of Fig. 4B. Shown in the right panel are bar graphs for the MFI of each group. The 6H8-HSP90 complex was found to disappear from the cell surface (to enter into cells) in a very short time of about 10 minutes.

Furthermore, immunostaining was performed to compare the behavior of HSP90 with the behaviors of the MHC class I molecule H-2K^{b} and the MHC class II molecule I-A^{b}. First, DC2.4 cells were incubated in the presence of the biotinylated 6H8 antibody and the FITC-labeled anti-H-2K^{b} antibody or anti-I-A^{b} antibody (Pharmingen, the antibody was used at a 100-fold dilution) in the same manner as the above. The cells were stained with PE-Cy5-streptavidin, returned to room temperature and incubated for 20 minutes, and then examined using a confocal microscope. Fig. 4C shows the results for HSP90 and H-2K^{b}; Fig. 4D shows the results for HSP90 and I-A^{b}. It was confirmed that cell surface HSP90 was internalized by the 6H8 antibody.

### Example 7

Described in this Example is the fact that it is possible to cross-present foreign antigen conjugated to the antibody of the present invention to CD8⁺ T cells.
The results of Example 6 show the possibility that the 6H8 antibody can be used as an adjuvant for efficiently introducing an antigen into cells. Hence, using ovalbumin (OVA) as a model antigen, the potential of the 6H8 antibody as an adjuvant was investigated.
Imject maleimide-activated OVA was obtained from Thermo Scientific (Rockford, USA). 1 ml of 6H8 mAb (5 mg/ml) (50 mM phosphate, 1 mM EDTA) was reacted with 10 µl of an SATA solution (6.5 mg of N-Succinimidyl S-acetylthioacetate dissolved in 500 µl of DMSO) at room temperature for 30 minutes. After three cycles of centrifugation and washing using a 30,000 MW cut-off centrifugal filter (Millipore), the reaction mixture was adjusted to a volume of 1 ml; 100 µl of 0.5 M hydroxylamine was added, and the reaction was allowed to proceed at room temperature for 2 hours. This was reacted with maleimide-activated OVA in a 1:1.5 ratio for 90 minutes, and 10 mM (final concentration) 2-ME was added to stop the reaction.
The reaction mixture of the 6H8 antibody and maleimidated OVA was subjected to size exclusion chromatography using the Superose 6 column (GE Healthcare), and the conjugate was separated from the unreacted protein. The size exclusion chromatography was performed using the Superose 6 column and the AKTA purifier (GE Healthcare), with the use of PBS for the mobile phase at a flow rate of 0.5 ml/min. Preparative collection was performed at 0.5 ml/fraction. Fig. 6A shows the chromatogram obtained. The operations above yielded fractions 1 to 50.

By performing immunoblotting using anti-mouse IgG-HRP (to detect the 6H8 antibody) and the biotinylated anti-OVA monoclonal antibody (to detect OVA), the molecular weight profile of each fraction was determined (Fig. 6B). It was confirmed that the fractions 15 to 19 contained a high molecular weight 6H8-OVA conjugate, and that the fraction 33 contained non-conjugated OVA but did not contain the 6H8 antibody.

A cross-presentation assay was performed using each fraction as an antigen. DC2.4 cells were pulsed with 5 µg/ml (30 µg/ml, only for the fraction 33) of each fraction or 1 mg/ml of free OVA for 3 hours. After fixation using para-formaldehyde, DC2.4 cells (1x10⁴ cells/well) were co-cultured in a 96-well round-bottomed plate with OT-I CD8⁺ T cells [1x10⁵ cells/well: splenocytes of an OT-I mouse were recovered, and CD11b-positive cells were removed according to a standard protocol using BD IMag anti mouse CD11b magnetic particles DM (BD Biosciences Pharmingen, 558013); next, CD8a-positive cells were purified according to a standard protocol using BD IMag anti mouse CD8a particles DM (BD Biosciences Pharmingen, 551516); the cells obtained were used as OT-I CD8⁺ T cells]. 24 to 72 hours later, the culture supernatant was collected and assayed to determine the IFNγ content. As shown in Fig. 6C, DC2.4 cells pulsed with the fractions 15 to 21 containing a high molecular weight complex (i.e., 6H8-OVA conjugate) efficiently induced IFNγ production by CD8⁺ T cells. The amounts of proteins in these fractions used to pulse the cells (the total amounts of the 6H8 antibody and maleimidated OVA) were less than 5 µg/ml in all cases, which were less than 1/200 of the amount of OVA contained in the positive control (OVA).

Furthermore, using DC2.4L cells lacking cell surface HSP90 (a line detectable with 6H8 antibody, and showing decreased expression levels of cell surface HSP90 and H-2K^{b}; obtained during cultivation of DC2.4 and established by passage culture), the specificity of 6H8-OVA for cell surface HSP90 was examined. DC2.4 and DC2.4L cells were incubated along with the fractions 15 to 19 containing 6H8-OVA (mixture) (30 µg/ml), after which the cells were stained with the biotinylated anti-OVA monoclonal antibody and PE-Cy5 streptavidin, and analyzed by FACS. As shown in Fig. 6D, DC2.4 cells expressing cell surface HSP90 (left upper panel) were recognized by the anti-OVA monoclonal antibody (right upper panel). As expected, DC2.4L cells lacking cell surface HSP90 (left lower panel) were not recognized by the OVA monoclonal antibody (right lower panel). These results demonstrate that 6H8-OVA works as an HSP90-specific antibody even when conjugated with OVA.

Next, DC2.4 cells were pulsed with a series of doses of 6H8-OVA (fractions 15 to 19) and free OVA (fraction 33) and incubated along with OT-I CD8⁺ T cells and OT-II CD4⁺ T cells [splenocytes of an OT-II mouse were recovered, and CD11b-positive cells were removed according to a standard protocol using BD IMag anti mouse CD11b magnetic particles DM (BD Biosciences Pharmingen, 558013); next, CD4-positive cells were purified according to a standard protocol using BD IMag anti mouse CD4 particles DM (BD Biosciences Pharmingen, 551539); the cells obtained were used as the OT-II CD4⁺ T cells], and the amount of IFNγ secreted from T cells was measured, whereby the cross-presentation potential of 6H8-OVA was evaluated. As a result, 6H8-OVA was at least 100 times more effective on OT-I CD8⁺ T cells than free OVA (Fig. 6E, upper panel). In contrast to the activation of OT-1 CD8⁺ T cells, the antigen presentation to OT-II CD4⁺ T cells was weak, although it was better than free OVA (Fig. 6E, lower panel).

### Example 8

Described in this Example is the fact that the cross-presentation of a foreign antigen conjugated to the antibody of the present invention is mediated by cell surface HSP90.
Pellets of DC2.4 cells (1.5x10⁶ cells) were incubated with 20 µg/ml of each antibody [6H8D2 (6H8), 2A6E9 (2A6) or 4B6G12 (4B6)] at 37°C with 5% CO₂ for 15 minutes. Subsequently, 6H8-OVA was added to a concentration of 3 or 1 µg/ml, and the pellets were incubated at 37°C with 5% CO₂ for 3 hours. After being washed to remove the unreacted antibody and 6H8-OVA, the cells were fixed in 0.5% para-formaldehyde. These cells (1x10⁴ cells) were co-cultured with 1x10⁵ OT-I CD8⁺ T cells in a 96-well round-bottomed plate at 37°C with 5% CO₂; the supernatant was recovered as appropriate and assayed for IFNγ according to a standard ELISA method.
As shown in Fig. 7, the cross-presentation effect of 6H8-OVA was attenuated by treating the DC2.4 cells with 6H8 in advance, but was not suppressed by the 2A6E9 or 4B6G12 antibody at all. These results show that the cross-presentation was inhibited as a result of the binding of the 6H8 antibody to cell surface HSP90. Hence, the cross-presentation of the foreign antigen conjugated to the antibody of the present invention is mediated by cell surface HSP90.

### Example 9

Described in this Example is the fact that the 6H8-OVA conjugate possesses a CD8⁺ T cell activation potential in vivo.
First, CFSE-labeled OT-I CD8⁺ T cells were generated as follows: A 10 mM CFSE stock solution in DMSO was diluted with PBS to prepare a 10 µM CFSE solution. A pellet of splenocytes prepared in a 15-ml tube was thoroughly loosened by tapping, after which 2 ml of the 10 µM CFSE solution was added thereto, and the mixture was gently vortexed. After being allowed to stand at room temperature for 5 minutes, the cells were washed twice with PBS (5 ml).
The CFSE-labeled OT-I CD8⁺ T cells were transplanted to recipient mice from the tail vein. The recipient mice used were a wild-type B6 mouse (WT B6) and a TAP1-deficient mouse [TAP1 KO (purchased from Jackson Laboratory, U.S.A.)]. At the same time, 3 µg of the fractions 15 to 19 (mixture), 3 µg of the fraction 33, or a mixture of the fraction 33 (3 µg) and the free 6H8 antibody (3 µg) was injected. Three days later, splenocytes of each recipient mouse were collected and examined for proliferation of the transplanted cells. As a result, in the mouse receiving the fractions 15 to 19 (mixture), proliferation of CD8⁺ T cells was confirmed (Fig. 8A). Meanwhile, in the TAP1-deficient mouse, the fractions 15 to 19 (mixture) did not induce the proliferation of CD8⁺ T cells at all (Fig. 8B). These results demonstrate that the cross-presentation of 6H8-OVA is dependent on the TAP molecule.

A mixture of the fractions 15 to 19 containing the 6H8-OVA conjugate was injected into recipient mice at a series of doses, and the proliferation of OT-1 CDB⁺ T cells was examined in the same manner as the above. As a result, higher doses of the mixture more potently promoted the proliferation of CD8⁺ T cells, although this effect was observed even at 0.1 µg (upper panel of Fig. 8C). Meanwhile, the promotion of the division of OT-II CD4⁺ T cells required a higher dose of the 6H8-OVA conjugate (lower panel of Fig. 8C). The results above show that the OVA conjugated to the 6H8 antibody was delivered to the MHC class I antigen presentation pathway and presented to CD8⁺ T cells, resulting in the activation of the CD8⁺ T cells.

Furthermore, since the division of OT-I CD8⁺ T cells does not necessarily mean in vivo cross-priming, it was determined whether cytotoxic T cells specific for the major OVA₂₅₇₋₂₆₄ epitope were generated by immunizing mice with 6H8-OVA (3 µg per mouse). The mice were immunized twice at a 1-week interval. One week after second immunization, splenocytes were cultured in the presence of the OVA₂₅₇₋₂₆₄ peptide for 5 days. The resulting cytotoxic T cells were monitored by tetramer staining [T cells were stained using T-Select H-2K^{b} OVA Tetramer-SIINFEKL-PE (MBL, TS5001-1, amount used = 1 test/tube) at room temperature for 30 minutes], and standard ⁵¹Cr release assay [2x10⁶ each of mouse tumor cells E.G7, supplied by Dr. Michael Bevan at the University of Washington, U.S.A. (see Cell vol.54:777-785, 1988), and EL4 (purchased from ATCC) were labeled with 50 microcuries of sodium ⁵¹Cr, and used as the target cells; the cytotoxic T cells were added to 5x10³ target cells as indicated in Fig. 8E; after cultivation for 4 hours, free ⁵¹Cr in the supernatant was measured to calculate the cytotoxic activity; the EL4 cells with or without pulsing with the peptide OVA₂₅₇₋₂₆₄ were prepared]. As is evident from the results of FACS analysis, immunization with 6H8-OVA produced tetramer-positive cells (Fig. 8D), and cytotoxicity to OVA₂₅₇₋₂₆₄ epitope was also observed (Fig. 8E). These results show that immunization with a complex of 6H8 and an antigen can produce antigen-specific cytotoxic T cells.

Hence, the vaccine of the present invention allows an antigen conjugated to the antibody of the present invention to be cross-presented to CD8⁺ T cells (Fig. 23). Provided that a tumor antigen is chosen as the antigen, the vaccine of the present invention could be effective in suppressing tumor metastasis.

### Example 10

Described in this Example is the fact that the 6H8 antibody enhances the activity of the 6H8-OVA conjugate of inducing the proliferation of OT-I CD8⁺ T cells in vivo.
The 6H8 antibody or IgG2a [normal mouse IgG2a (nmIgG2a), 20 µg] was diluted with 200 µl of PBS and administered to B6 mice from the tail vein. As a control, a group of animals that received PBS alone was also prepared.
Subsequently, CFSE-labeled OT-I CD8⁺ T cells (1.5x10⁶ cells) were suspended in 200 µl of RPMI and administered from the tail vein. Concurrently, 6H8-OVA (0.3 µg) diluted with 200 µl of PBS was administered from the tail vein. As a control, a group of animals that received PBS alone was also prepared.
72 hours later, splenocytes were recovered from each recipient mouse, and decay of CFSE intensity were measured using FACS Calibur (BD Biosciences) to confirm the proliferation of transferred cells.
As shown in Fig. 9, when the 6H8 antibody was administered in advance, the effect of promotion of the proliferation of CD8⁺ T cells by 6H8-OVA was enhanced.

### Example 11-1

Described in this Example is the fact that the Alexa 488-labeled 6H8 antibody administered to mice accumulates in CD11b⁺ monocytes in the spleen.
First, the Alexa 488-labeled 6H8 antibody was generated according to a standard protocol using the Alexa Fluor 488 Protein Labeling Kit (Invitrogen, Molecular probes, A10235).
The Alexa 488-labeled 6H8 antibody (20 µg) was diluted with 200 µl of PBS and administered to B6 mice from the tail vein. Three hours later, the spleen was recovered from each recipient mouse and treated with collagenase as follows: An RPMI (10 ml) containing collagenase (400 U/ml, Wako, 032-10534) and DNase (15 µg/ml, Roche, 10401600) was added to a 6-cm dish, and the spleen was thoroughly loosened therein. Subsequently, the dish was incubated at 37°C with 5% CO₂ for 10 minutes. After stirring the mixture with a Pasteur pipette, EDTA was added to obtain a final concentration of 5 mM, and the dish was further incubated at 37°C with 5% CO₂ for 5 minutes. Subsequently, the dish was washed twice with RPMI (5 ml).
After collagenase treatment, erythrocytes were disrupted. The sample was blocked while cooling on ice using mouse IgG (SIGMA, I5381, 10 µg/ml) for 30 minutes (100 µl/10⁶ cells). Subsequently, the cells were stained using various antibodies [PE-Cy5 conjugate Anti-Mouse CD11b: (eBiosciences, 15-0112-81, 0.2 µg/ml), PE conjugate Anti-mouse/human CD45(B220): (eBiosciences, 12-0452-81, 1 µg/ml), PE anti-mouse CD11c(HL3) (Integrin αx chain): (BD Pharmingen, 553802, 1 µg/ml)] while cooling on ice for 30 minutes (100 µl/10⁶ cells), and analyzed using FACS Calibur.
While the 6H8 antibody is thought to bind to activated dendritic cells, macrophages and the like in vivo, the administered Alexa 488-labeled 6H8 antibody bound particularly to CD11b⁺ monocytes, as shown in Fig. 10A. Therefore, there is a possibility that 6H8 antibody-binding monocytes as antigen-presenting cells directly activate T cells, or that the antigen is first transferred from monocytes to dendritic cells, which dendritic cells then activate T cells.

### Example 11-2

Described in this Example is the fact that the Alexa 647-labeled 6H8 antibody administered to mice accumulates in dendritic cells (CD11c-positive), particularly in CD8α-positive dendritic cells, in the spleen.
First, the Alexa 647-labeled 6H8 antibody was generated according to a standard protocol using the Alexa Fluor 647 Protein Labeling Kit (Invitrogen, Molecular probes, A20173).
The Alexa 647-labeled 6H8 antibody (30 µg) was diluted with 200 µl of PBS and administered to B6 mice from the tail vein. 1.5 hours later (or 3 hours later), the spleen was recovered from each recipient mouse and treated with collagenase as follows: An RPMI containing collagenase (400 U/ml, Wako, 032-10534) and DNase (15 µg/ml, Roche, 10401600) (10 ml) was added to a 6-cm dish, and the spleen was thoroughly loosened therein. Subsequently, the dish was incubated at 37°C with 5% CO₂ for 10 minutes. After stirring using a Pasteur pipette, EDTA was added to obtain a final concentration of 5 mM, and the dish was further incubated at 37°C with 5% CO₂ for 5 minutes. Subsequently, the dish was washed twice with RPMI (5 ml).
After collagenase treatment, erythrocytes were disrupted. The sample was blocked using mouse IgG (SIGMA, I5381, 10 µg/ml) while cooling on ice for 30 minutes (100 µl/10⁶ cells). Subsequently, the cells were stained using various antibodies [PE-Cy5 conjugate Anti-Mouse CD11b: (eBiosciences, 15-0112-81, 0.2 µg/ml), PE conjugate Anti-mouse/human CD45(B220): (eBiosciences, 12-0452-81, 1 µg/ml), PE anti-mouse CD11c (HL3) (Integrin αx chain): (BD Pharmingen; 553802, 1 µg/ml), PE anti-mouse CDBα (Ly-2) (Integrin αx chain): (BD Pharmingen, 12-0081, 1 µg/ml)] while cooling on ice for 30 minutes (100 µl/10⁶ cells) and analyzed using FACS Canto.
The 6H8 antibody is thought to bind to activated dendritic cells, macrophages and the like in vivo; the region corresponding to the R2 region in Fig. 10A was examined in detail. As a result, as shown in Fig. 10B, the Alexa 647-labeled 6H8 antibody administered was incorporated by dendritic cells (CD11c-positive). As shown in Fig. 10C, the antibody bound particularly to CD8a-positive dendritic cells. Therefore, there is a possibility that CDBα-positive dendritic cells serve as antigen-presenting cells to activate T cells.

### Example 12

Described in this Example is that the monoclonal antibody of the present invention promotes the cross-presentation mechanism mediated by an Fc receptor, that is, it is also useful as an excellent adjuvant.
The proliferation of CD8⁺ T cells in the presence of the 6H8 antibody in its free form was examined in the same manner as the in vivo cross-presentation assay described in Example 9, as described below.
(1) CFSE-labeled CD8⁺ T cells of an OT-I/CD45.1 mouse were transferred to recipient mice from the tail vein, with 6H8-OVA [or 6H8 (F(ab')₂)-OVA, or OVA, or IgG2a-OVA] (0.3 µg) and the free 6H8 antibody [or 6H8 (F(ab')₂) or IgG2a] (30 µg) administered from the tail vein concurrently.
(2) 72 hours later, the spleen was recovered from each mouse, and 2x10⁶ splenocytes were stained with the PE-CD45.1 antibody in a 96-well round-bottomed plate for 30 minutes (while cooling on ice).
(3) The CFSE intensity of CD45.1-positive cells (OT-I CD8⁺ T cells) was determined using FACS Canto (BD).
The results are shown in Fig. 11. When 0.3 µg of 6H8-OVA was administered, adoptively transferred OT-I CD8⁺ T cells exhibited a slight proliferation reaction. When the 6H8 antibody or 6H8 (F(ab')₂) antibody was administered concurrently, however, the proliferation reaction of the OT-I CD8⁺ T cells was remarkably enhanced. In contrast, when 6H8 (F(ab')₂)-OVA or OVA was administered, an effect of enhancing the proliferation reaction of the OT-I CD8⁺ T cell by the 6H8 antibody was not noted. When IgG2a-OVA was administered, an enhancing effect by the 6H8 antibody was noted.
The results above lead to the conclusion that the binding of the monoclonal antibody of the present invention to dendritic cells promotes the cross-presentation mechanism via an Fc receptor. The results also suggest that stimulation with the monoclonal antibody of the present invention intersects with stimulation with the Fc receptor; for example, it is considered that a common signal cascade is activated via the FcRγ subunit (Figs. 12A and B).

### Example 13

Described in this Example is the fact that the expression of HSP90 on the cell surface is dependent on FcRγ.
To verify the model shown in Fig. 12, the following experiments were performed using GM-CSF-dependent BMDC.

### 1. Expression of cell surface HSP90:

(1) A bone marrow-derived dendritic cells (BMDCs) were established from a normal mouse and an FcRγ KO mouse (supplied by Professor Akira Shibuya, Immunology, Life System Medical Sciences, Majors of Medical Sciences, Graduate School of Comprehensive Human Sciences, University of Tsukuba) as described below. Bone marrow was recovered from a mouse femur and tibia, and 1x10⁶ cells each were cultured in a 24-well plate using 2 ml/well of a complete RPMI containing 20 ng/ml recombinant mouse GM-CSF (R&D). Half of the medium was exchanged for a fresh medium on day 3 of cultivation, and the cells obtained on day 5 of cultivation were used as the BMDC.
(2) BMDCs at Day 5 induced in a GM-CSF-dependent manner was stained with biotin-6H8 (2.5 µg/ml) and streptavidin-APC (0.2 µg/ml), and its fluorescence was determined using FACS Canto (BD).
The results are shown in Fig. 13. Expression of cell surface HSP90 (in Fig. 13, HSP90) was observed in normal mouse-derived dendritic cells (WT B6) but disappeared completely in KO mouse-derived dendritic cells (FcRγ KO).

### 2. In vitro cross-presentation assay:

(1) A pellet of 1x10⁶ BMDC cells at Day 5 induced in a GM-CSF-dependent manner were prepared in a 15-ml tube. After adding 400 µl of an antigen (6H8-OVA, OVA, or OVA₂₅₇₋₂₆₄) thereto, the tube was incubated with 5% CO₂ at 37°C for 3 hours. The cells were fixed in 0.5% para-formaldehyde, after which the cells were washed with 0.1 M glycine and then with RPMI; thereafter, the density was adjusted to 1x10⁵ cells/ml.
(2) Concurrently, OT-I CD8⁺ T cells were purified and the density was adjusted to 1x10⁶ cells/ml.
(3) After being treated as described above, the BMDC (1x10⁴ cells) and the OT-I CD8⁺ T cells (1x10⁵ cells) were co-cultured in a 96-well round-bottomed plate.
(4) The supernatant was recovered as appropriate and assayed for the amount of produced IFNγ using ELISA.
The results are shown in Fig. 14. It was shown that the cross-presentation potential of FcRγ KO mouse-derived dendritic cells (FcRγ KO) for 6H8-OVA was significantly decreased as compared with normal mouse-derived dendritic cells (WT B6). Meanwhile, for OVA or OVA₂₅₇₋₂₆₄, KO mouse-derived dendritic cells exhibited a cross-presentation potential equivalent to that of normal mouse-derived dendritic cells.
Furthermore, the in vivo proliferation reaction of OT-I CD8-positive cells also was significantly decreased in the FcRγ KO mouse (data not shown).
The results above showed that the expression of HSP90 on the cell surface is dependent on FcRγ, and that FcRγ plays a key role in the functioning of the vaccine of the present invention (Fig. 24).

### Example 14

Described in this Example is the fact that differentiation into memory T cells is induced in vivo by administration of the vaccine of the present invention.
Memory T cells are T cells involved in immune memory. After being produced in a body of a living organism sensitized once by a certain antigen, they stay in the body for a long time, and quickly cause immune responses in response to restimulation with the same antigen. Therefore, the induction of differentiation into memory T cells by administration of a vaccine means that the effect of the vaccine persists for a longer time.
To examine the possibility that memory T cells are induced by administration of the vaccine of the present invention, experiments were performed as described below.
(1) 2x10⁶ CD8⁺ cells of an OT-I/CD45.1 mouse were transferred to a C57BL/6 mouse from the tail vein, with 3 µg of 6H8-OVA or PBS administered from the tail vein concurrently.
(2) 96 hours later, the mouse spleen was recovered, and 2x10⁶ splenocytes were stained with the following antibodies in a 96-well round-bottomed plate for 30 minutes (while cooling on ice).
   - PE-anti-mouse/human CD44 (eBioscience: Cat No. 12-0041), concentration: 1 µg/ml, amount used: 100 µl. Biotin-anti-CD45.1 (eBioscience: 13-0453), concentration: 2.5 µg/ml, amount used: 100 µl.
(3) After being washed twice with PBS, the cells were stained with the following antibody for 30 minutes (while cooling on ice).
   - Streptavidin-APC (BioLegend: 405207), concentration: 0.2 µg/ml, amount used: 100 µl.
(4) After being washed twice with Annexin-binding buffer, the cells were stained with FITC-Annexin V using the Annexin V FITC reagent for 10 minutes (while cooling on ice).
   - Annexin-binding buffer (HEPES 10 mM, NaCl 150 mM, KCl 5 mM, MgCl₂ 1 mM, CaCl₂ 1.8 mM)
   - Annexin V FITC reagent (MBL: BV-1001-5), concentration: 5-fold dilution of stock solution, amount used: 500 µl (5) Fluorescence was determined using FACS Canto (BD).
The results are shown in Fig. 15. Adoptively transferred OT-I CD8⁺ T cells were activated by administration of 6H8-OVA, and the expression level of CD44 was increased as compared with the group that did not receive 6H8-OVA. This shows that administration of 6H8-OVA caused transition to memory T cells. In both of the group that received 6H8-OVA and the group that did not received, a part of cells became Annexin V-positive cells, suggesting that they had undergone apoptosis. The right panel of Fig. 15 shows the results of a control experiment wherein the function of the Annexin V used as a reagent was confirmed using DC2.4 cells in which apoptosis was induced by UV irradiation.
The results above show that differentiation into memory T cells was induced by the vaccine of the present invention.

### Example 15

Described in this Example is induction of cytokine production from bone marrow-derived dendritic cells by stimulation with the 6H8 antibody immobilized on a solid phase.
To determine the influence of the 6H8 antibody on cytokine production from bone marrow-derived dendritic cells, experiments were performed as described below.
(1) 50 ml of the 6H8 antibody diluted in PBS (10 mg/ml) was added to a 96-well flat plate (Corning Incorporated, product number 3596), and the plate was treated at 4°C overnight.
(2) The plate was washed twice with 200 ml of PBS to remove the excess 6H8 antibody.
(3) On day 5 after induction, GM-CSF-dependent bone marrow-derived dendritic cells were added to the plate from (2) at 1x10⁵/well and cultured in an incubator at 37°C with 5% CO₂.
(4) 12 hours after the start of cultivation, the supernatant was recovered; the amounts of cytokines and chemokines in the supernatant were determined according to a standard protocol using the Bio-Plex suspension array system with 23-Plex Panel (Bio-rad, 171-F11241).
As seen in the results in Fig. 16, production of IL-1α, IL-1β, IL-2, IL-4, IL-9, IL-10, IL-12(p40), IL-12(p70), IL-13, G-CSF, GM-CSF, KC, MCP-1, MIP-1α, MIP-1β, RANTES and the like was induced by stimulation with the 6H8 antibody. The results above showed that the vaccine of the present invention is capable of stimulating and activating dendritic cells and possesses an excellent characteristic as an adjuvant.

### Example 16

In this Example, the results of examination of the specific CTL induction potential of the 6H8-antigen at low concentrations, and the results of comparison with the specific CTL induction potential in the case where the antigen was used along with a preexisting adjuvant are shown.
Mice were immunized twice with 6H8-OVA or IFA+OVA at a 1-week interval. For 6H8-OVA, the specified amount of 6H8-OVA was diluted in 200 µl of PBS and administered from the tail vein. For IFA+OVA, an emulsion was prepared so that the specified amount of OVA₂₅₇₋₂₆₄ peptide would be contained in the 200 µl dose, and subcutaneously administered. The emulsion was prepared by mixing the required amount of the OVA₂₅₇₋₂₆₄ peptide in PBS in advance, and adding an equal amount of IFA (SIGMA: F5506) thereto.
Seven days after final immunization, the spleen of each recipient mouse was collected and finely loosened using tweezers, and splenocytes were recovered. A pellet of splenocytes was prepared in a 15-ml tube, and 2 ml of an FCS-free complete RPMI containing the OVA₂₅₇₋₂₆₄ peptide (final concentration 10⁻⁵ M) was added thereto. With the lid of the tube loosened, the tube was allowed to stand in an incubator at 37°C with 5% CO₂ for 1 hour. Subsequently, the culture broth RPMI (containing 10% FCS) was added to adjust the total volume to 20 ml, and 2 ml each was put into a 24-well plate.
After 5 days of cultivation, the cells were recovered and stained using the H-2K^{b}+OVA₂₅₇₋₂₆₄-specific tetramer (MBL: TS-5001-1) and FITC-anti-mouse CD8a (eBioscience: 11-0081), and induction of specific CTL was confirmed using FACS Canto. The staining was achieved according to the instructions for the H-2K^{b}+OVA₂₅₇₋₂₆₄-specific tetramer.
The results are shown in Fig. 17. It was shown that 6H8-OVA is as effective as, or more effective than, IFA+OVA in CTL induction.

### Example 17

Described in this Example is the fact that inflammation due to administration of dead cells of Propionibacterium acnes (P. acnes) induces the expression of HSP90 and CD64 (Fcγ receptor) on the dendritic cell membrane surface.
P. acnes in the amounts shown in Fig. 18 was diluted in 200 µl of PBS and administered to mice from the tail vein. Six days (experiment 1) or 0 to 10 days (experiment 2) after administration, the spleen of each recipient mouse was collected, and subjected to collagenase treatment and Gey's solution treatment as follows to recover splenocytes.
The collagenase treatment is described in detail below.
One spleen was finely loosened in a 6-cm dish containing 10 ml of a collagenase solution using tweezers and the like and allowed to stand at 37°C in an incubator for 15 minutes. EDTA was added thereto to a final concentration of 5 mM; the mixture was pipetted and further allowed to stand at 37°C in an incubator for 5 minutes, after which the mixture was passed through a mesh and transferred to a 15-ml tube. Collagenase solution: 400 U/ml collagenase (Wako, 032-10534) and 15 µg/ml DNase were dissolved in RPMI. A fresh supply was prepared just before use.
The Gey's treatment is described in detail below.
A pellet of cells was prepared in a 15-ml tube and thoroughly loosened by tapping. After 2 ml of the Gey's solution was added thereto, the tube was allowed to stand on ice for 3-5 minutes. Subsequently, 10 ml of PBS was added, the mixture was quickly centrifuged, and the Gey's solution was removed.
Gey's solution: The following liquids A-D were mixed in a ratio of 20% A, 5% B, 5% C, and 70% D.
Liquid A: NH₄Cl (3.5 g), KCl (0.185 g), Na₂HPO₄ (0.06 g), KH₂PO₄ (0.012 g), D-glucose (0.5 g), bromophenol red (5 mg)
Liquid B: MgCl₂•6H₂O (0.42 g), MgSO₄.7H₂O (0.14 g), CaCl₂ (0.34 g)
Liquid C: NaHCO₃ (2.25 g)
Liquid D: DW
The respective ingredients were dissolved in 100 ml of DW and autoclaved before use.
Subsequently, 2x10⁶ splenocytes were stained in a 96-well round plate and analyzed using FACS Canto. Blocking and antibody staining were achieved by allowing 100 µl per well of an antibody to stand on ice for 30 minutes. Between stainings, the plate was washed twice with 200 µl of PBS. The following antibodies were used: Mouse IgG (SIGMA: I5381, 100 µg/ml) (for blocking), FITC-anti mouse CD8a (eBioscience: 11-0081, 5 µg/ml), PE-anti mouse B220 (eBioscience: 12-0452, 1 µg/ml), PE-anti mouse CD64 (BioLegend: 139304, 1 µg/ml), PE-anti mouse CD86 (BioLegend: 105508, 1 µg/ml), PE-Cy5-anti mouse CD11b (eBioscience: 15-0112, 0.4 µg/ml), PE-Cy5-anti mouse CD4 (eBioscience: 15-0042, 0.4 µg/ml), APC-Streptavidin (BioLegend: 405207, 0.2 µg/ml), APC-Cy7-anti mouse CD11c (BioLegend: 117324, 2 µg/ml), and Biotin-6H8 (10 µg/ml). Splenomegaly was also evaluated (++++, severe splenomegaly; ++, moderate splenomegaly; +, mild splenomegaly; -, no splenomegaly).
As shown in Fig. 18, the expressions of HSP90 and CD64 (FCγ receptor) were enhanced in proportion to the amount of P. acnes administered and the time after administration.

### Example 18

Described in this Example are essential factors for changes in the expression of HSP90 and CD64 on the spleen dendritic cell membrane surface by administration of P. acnes.
<Experiment 1> 500 µg of P. acnes diluted in 200 µl of PBS was administered to C57BL/6 mice, MyD88/TRIF knockout (KO) mice, IFNγ KO mice, and IFNα/β receptor KO mice from the tail vein. Six days after administration, the spleen of each recipient mouse was collected and analyzed in the same manner as Example 17.
<Experiment 2> The same analysis as Experiment 1 was performed using C57BL/6 mice, IL-18 KO mice, and Fc receptor γ-chain (FcRγ) KO mice.
The results are shown in Fig. 19. The results of Experiment 1 demonstrated that the activation of dendritic cells (expression of HSP90 and CD64) by administration of P. acnes was dependent on MyD88 and also dependent on IFNγ. Meanwhile, this activation did not depend on IFNa/β. The results of Experiment 2 demonstrated that the activation was not dependent on IL-18 but did depend on the FcRγ subunit.

### Example 19

Described in this Example are differences in the expression of membrane surface HSP90 and CD64 due to administration of P. acnes in various cell populations of C57BL/6 mice.
500 µg of P. acnes diluted in 200 µl of PBS was administered to C57BL/6 mice from the tail vein. Six days after the administration, the spleen of each recipient mouse was collected, and splenocytes were recovered via the above-described collagenase treatment and Gey's solution treatment. Subsequently, 2x10⁶ splenocytes were subjected to a blocking treatment using a mouse IgG antibody (100 µg/ml) in a 96-well round plate, after which they were stained with various antibodies. Blocking and antibody staining were achieved by allowing the cells to stand on ice for 30 minutes using 100 µl per well of each antibody. Between stainings, the plate was washed twice with 200 µl of PBS. The antibodies used for the staining are as follows (details of those mentioned above are not described here): <dendritic cells/macrophages> PE-Cy5-anti mouse CD11b (eBioscience: 15-0112, 0.4 µg/ml), APC-Cy7-anti mouse CD11c, PE-anti mouse CD64, Biotin-6H8, APC-Streptavidin; <T cells> APC-Cy7-anti-mouse CD11c, FITC-anti mouse CD8a, PE-Cy5-anti-mouse CD4 (eBioscience: 15-0042, 0.4 µg/ml), PE-anti-mouse CD64, Biotin-6H8, APC-Streptavidin; <B cells> APC-Cy7-anti-mouse CD11c, PE-anti-mouse B220 (eBioscience: 12-0452, 1 µg/ml), Biotin-6H8, APC-Streptavidin. After the staining, the cells were analyzed using FACS Canto.
Fig. 20 shows the expression of HSP90 (6H8)/CD64 in living cells in each cell population by means of two-dimensional dot plots. In the CD11c^{high}/CD11b(-) population (D), neither HSP90 nor CD64 was expressed. Meanwhile, both HSP90 and CD64 were expressed in CD11c^{high}/CD11b^{middle}(C) and CD11c^{middle}/CD11b^{high} (A and B) dendritic cells. In T cells and B cells, neither HSP90 nor CD64 was expressed. These findings demonstrated that the expression of HSP90 on the cell membrane surface due to administration of P. acnes is limited to antigen-presenting cells.

### Example 20

Described in this Example is an analysis of the 6H8 antibody gene.

### 1. Mouse antibody (IgG) sequence-specific RT reaction

Using as a template a total RNA prepared from a hybridoma that produces the 6H8 antibody (accession number FERM BP-11222) according to a conventional method, a cDNA was synthesized using a mouse antibody (IgG) heavy-chain (H-chain)-specific primer [H-RT1: TCCAKAGTTCCA (SEQ ID NO:7). Likewise, another cDNA was synthesized using a light-chain (L-chain)-specific primer [L-RT1: GCTGTCCTGATC (SEQ ID NO:8)]. An RT reaction was carried out using the SMARTer™ RACE cDNA Amplification Kit (Clontech Cat. No. 634924) under the conditions shown below, according to the manufacturer's instruction manual.
(1) Using a mixture of 0.5 µg of the total RNA, 1 µl of H-RT1 or L-RT1 (12 µM), and 3.75 µl of dH₂O, the reaction was carried out at 70°C for 3 minutes and at 42°C for 2 minutes.
(2) 1 µl of SMARTer II A Oligonucleotide (12 µM), 1 µl of DTT (20 mM), 1 µl of dNTP Mix (10 mM each), 2 µl of 5xFirst-Strand Buffer, 0.25 µl of RNase Inhibitor (40 U/µl), and 1 µl of SMARTScribe™ Reverse Transcriptase (100 U/µl) were added to the reaction liquid, and the reaction was carried out at 42°C for 90 minutes and at 70°C for 10 minutes.
(3) After 50 µl of Tricine-EDTA buffer was added to stop the reaction, the reaction mixture was stored at -20°C.

### 2. Mouse antibody (IgG) sequence-specific RACE PCR reaction

A 5'RACE PCR analysis was performed using the SMARTer™ RACE cDNA Amplification Kit.
(1) An RACE PCR reaction was carried out using a mouse antibody (IgG)H-chain-specific primer as a reverse primer and the UPM (Universal primer mix) contained in the kit as a forward primer, with the cDNA synthesized in the section 1 above (synthesized using H-chain-specific primer) as a template. Likewise, another RACE PCR reaction was carried out using an L-chain-specific primer with the cDNA (synthesized using L-chain-specific primers) as a template. The PCR enzyme used was PrimeSTAR (Takara Bio Inc.).
   The PCR reaction was carried out according to the protocol attached to the above-described kit.
(2) Generation of PCR products of expected sizes was confirmed by agarose gel electrophoresis. The PCR products were named SYN3025H and SYN3025L and used for the analysis after gel purification.

### 3. Cloning and base sequence analysis

(1) The gel-purified PCR products (SYN3025H, SYN3025L) were ligated to the cloning plasmid pMD20-T (Takara Bio Inc.).
(2) Transformation was carried out according to a conventional method; 48 clones were obtained for each PCR product.
(3) The sequences of the inserts contained in the clones obtained were analyzed according to a conventional method. The sequencing reaction was carried out using the BigDye Terminators v3.1 Cycle Sequencing Kit (ABI) and the ABI3730 Sequencer (ABI), according to the manufacturer's protocol.
(4) Results of base sequence analyses for 48 clones of each of the H-chain and the L-chain, as well as base sequences generated by excluding vector regions and regions of low reliability therefrom were obtained.

### 4. Evaluation of the results

Next, the following analyses were performed using the base sequences obtained in 3-(4).

### (1) Classification of the obtained sequences and obtainment of consensus sequences

The base sequences of the H-chain and the L-chain were classified based on the homology. Homology comparison was performed using the DNA sequence assembly software SEQUENCHER™ [Gene Codes; Windows (registered trademark) version]. As a result, one contig was obtained for the H-chain, and three contigs were obtained for the L-chain (some sequences did not constitute a contig). Consensus sequences were obtained from the resulting contigs.

### (2) Candidate sequence for the gene of interest

From the consensus sequences and the sequences that did not form a contig, sequences deemed candidates for the gene of interest were selected. Here, all the sequences that have a methionine residue, without containing a stop codon, upstream of the amino acid sequence of the antibody constant region gene were selected.

### (3) Deduction of amino acid sequences

Judging from the number of contig-forming sequences out of the candidate sequences and the gene lengths estimated for the resulting sequences, the major contigs of the H-chain and the L-chain were considered with a high probability to be the sequences of interest, respectively. Hence, the amino acid sequences encoded by the consensus sequences of the respective major contigs were determined to be the amino acid sequences of the H-chain and the L-chain.
The gene sequences and amino acid sequences of the variable regions of the 6H8 antibody H-chain and L-chain obtained as described above are shown in Figs. 21 and 22, respectively. The gene sequence and amino acid sequence of the H-chain variable region are shown in SEQ ID NOs:9 and 10, respectively. The gene sequence and amino acid sequence of the L-chain variable region are shown in SEQ ID NOs:11 and 12, respectively. In SEQ ID NO:10, the codon that encodes the second methionine is deemed to be the start codon; according to the numbering by Kabat et al. (Kabat, E.A., et al., Sequences of Proteins of Immunological Interest, 5th ed., 1991, Bethesda: US Dept. of Health and Human Services, PHS, NIH), the positions 17 to 35 correspond to the leader sequence, the positions 66 to 70 correspond to CDR1, the positions 85 to 101 correspond to CDR2, and the positions 134 to 141 correspond to CDR3. Likewise, in SEQ ID NO:12, the positions 1 to 19 correspond to the leader sequence, the positions 43 to 58 correspond to CDR1, the positions 74 to 80 correspond to CDR2, and the positions 113 to 121 correspond to CDR3.

### Industrial Applicability

The antibody of the present invention can be used, for example, to prepare the vaccine of the present invention, to deliver a compound possessing a biological activity, and to specifically detect cells expressing cell surface HSP90. Therefore, the antibody of the present invention is useful in treating a disease that can be treated using a vaccine, treating a disease that can be treated by delivering a drug into cells, and analyzing cells expressing cell surface HSP90 such as antigen-presenting cells. Furthermore, the antibody of the present invention can be used as an adjuvant to increase the efficiency of immunity induction with a vaccine and the like, and is useful in reducing the burden on the patient in the treatment of a disease.

This application is based on US provisional patent application No. 61/323,578 (filing date: April 13, 2010), the contents of which are hereby incorporated in its entirety.

## Claims

1. A monoclonal antibody that binds to an epitope comprising an amino acid sequence selected from the amino acid sequence VX₁X₂EX₃PPLEGDX₄ (wherein each of X₁ to X₄, which may be identical to or different from each other, represents an arbitrary amino acid) (SEQ ID NO:1) or the amino acid sequence HX_{S}IX₆ETLRQKAE (wherein each of X₅ to X₆, which may be identical to or different from each other, represents an arbitrary amino acid) (SEQ ID NO:2), and that recognizes cell surface HSP90.

2. The monoclonal antibody according to claim 1, wherein X₂ in the amino acid sequence of SEQ ID NO:1 is E or D.

3. The monoclonal antibody according to claim 1, wherein X₄ in the amino acid sequence of SEQ ID NO:1 is E or D.

4. The monoclonal antibody according to claim 1, wherein X₆ in the amino acid sequence of SEQ ID NO:2 is I or V.

5. The monoclonal antibody according to claim 1, wherein the epitope is an epitope comprising:
(1) the amino acid sequence of SEQ ID NO:1 wherein X₁ is T, X₂ is E, X₃ is M, and X₄ is D,
(2) the amino acid sequence of SEQ ID NO:1 wherein X₁ is P, X₂ is D, X₃ is I, and X₄ is E,
(3) the amino acid sequence of SEQ ID NO:2 wherein X₅ is S, and X₆ is I, or
(4) the amino acid sequence of SEQ ID NO:2 wherein X₅ is P, and X₆ is V.

6. The monoclonal antibody according to any one of claims 1 to 5, wherein the cell is an antigen-presenting cell.

7. The monoclonal antibody according to claim 6, wherein the antigen-presenting cell is an activated dendritic cell, a macrophage or a monocyte (mononuclear leukocyte).

8. The monoclonal antibody according to any one of claims 1 to 5, wherein the cell is a tumor cell.

9. The monoclonal antibody according to claim 8, wherein the tumor cell is of human origin.

10. A hybridoma having an accession number of FERM BP-11222 or FERM BP-11243.

11. A monoclonal antibody that binds to the same epitope as the epitope to which a monoclonal antibody produced by the hybridoma according to claim 10 binds, and that recognizes cell surface HSP90.

12. The monoclonal antibody according to any one of claims 1 to 9, which is produced by the hybridoma according to claim 10.

13. An antibody that recognizes cell surface HSP90, comprising at least one of heavy-chain CDR1 (the amino acid sequence shown by the positions 66 to 70 of SEQ ID NO:10), CDR2 (the amino acid sequence shown by the positions 85 to 101 of SEQ ID NO:10), CDR3 (the amino acid sequence shown by the positions 134 to 141 of SEQ ID NO:10), light-chain CDR1 (the amino acid sequence shown by the positions 43 to 58 of SEQ ID NO:12), CDR2 (the amino acid sequence shown by the positions 74 to 80 of SEQ ID NO:12), and CDR3 (the amino acid sequence shown by the positions 113 to 121 of SEQ ID NO:12).

14. An antibody that recognizes cell surface HSP90, comprising a heavy-chain variable region that comprises the amino acid sequence shown by the positions 36 to 185 of SEQ ID NO:10 and/or a light-chain variable region that comprises the amino acid sequence shown by the positions 20 to 177 of SEQ ID NO:12.

15. A method of producing a monoclonal antibody that recognizes cell surface HSP90, comprising the step of administering to an animal a polypeptide as an immunogen that comprises as an epitope an amino acid sequence selected from the amino acid sequence VX₁X₂EX₃PPLEGDX₄ (wherein each of X₁ to X₄, which may be identical to or different from each other, represents an arbitrary amino acid) (SEQ ID NO:1) or the amino acid sequence HX₅IX₆ETLRQKAE (wherein each of X₅ to X₆, which may be identical to or different from each other, represents an arbitrary amino acid) (SEQ ID NO:2).

16. A method of producing an antibody that recognizes cell surface HSP90, comprising the step of preparing a polypeptide that comprises at least one of heavy-chain CDR1 (the amino acid sequence shown by the positions 66 to 70 of SEQ ID NO:10), CDR2 (the amino acid sequence shown by the positions 85 to 101 of SEQ ID NO:10), CDR3 (the amino acid sequence shown by the positions 134 to 141 of SEQ ID NO:10), light-chain CDR1 (the amino acid sequence shown by the positions 43 to 58 of SEQ ID NO:12), CDR2 (the amino acid sequence shown by the positions 74 to 80 of SEQ ID NO:12), and CDR3 (the amino acid sequence shown by the positions 113 to 121 of SEQ ID NO:12).

17. A vaccine comprising a complex of an antibody that recognizes HSP90 on the cell surface and a target antigen.

18. The vaccine according to claim 17, wherein the antibody that recognizes HSP90 on the cell surface is the antibody according to any one of claims 1 to 9 and 11 to 14.

19. The vaccine according to claim 17 or 18, wherein the target antigen is a pathogen antigen or a tumor antigen.

20. A method of producing a vaccine, comprising the step of forming a complex of an antibody that recognizes HSP90 on the cell surface and a target antigen.

21. A method of inducing immunity, comprising the step of administering an effective amount of the vaccine according to any one of claims 17 to 19 to a subject in need thereof.

22. A method of treating or preventing a disease, comprising the step of administering an effective amount of the vaccine according to any one of claims 17 to 19 to a subject in need thereof.

23. A drug comprising a complex of an antibody that recognizes HSP90 on the cell surface and a substance possessing a biological activity.

24. The drug according to claim 23, wherein the antibody that recognizes HSP90 on the cell surface is the antibody according to any one of claims 1 to 9 and 11 to 14.

25. The drug according to claim 23 or 24, wherein the substance possessing a biological activity is a nucleic acid molecule.

26. The drug according to claim 25, wherein the nucleic acid molecule is an siRNA.

27. The drug according to claim 23 or 24, wherein the substance possessing a biological activity is an anticancer agent.

28. A method of treating or preventing a disease, comprising administering an effective amount of the drug according to any one of claims 23 to 27 to a subject in need thereof.

29. An adjuvant comprising a polypeptide that comprises the antigen-binding site of an antibody that recognizes HSP90 on the cell surface.

30. The adjuvant according to claim 29, wherein the antibody that recognizes HSP90 on the cell surface is the antibody according to any one of claims 1 to 9 and 11 to 14.

31. The adjuvant according to claim 29, wherein the antibody that recognizes HSP90 on the cell surface recognizes the amino acid sequence of SEQ ID NO:3 (VTEEMPPLEGDD) as an epitope.

32. The adjuvant according to claim 29, wherein the antigen-binding site of the antibody that recognizes HSP90 on the cell surface comprises the light-chain variable region in the antigen-binding site of the monoclonal antibody produced by the hybridoma whose accession number is FERM BP-11222.

33. A method of producing an adjuvant, comprising the step of mixing a polypeptide that comprises the antigen-binding site of an antibody that recognizes HSP90 on the cell surface with a pharmaceutically acceptable excipient or additive.

34. A method of inducing immunity, comprising the step of administering a vaccine that is incorporated into an antigen-presenting cell via binding to an Fc receptor and the adjuvant according to any one of claims 29 to 32 to a subject in need thereof.

35. The method of inducing immunity according to claim 34, wherein the antigen-presenting cell is a dendritic cell.

36. The method of inducing immunity according to claim 34, wherein the vaccine is the vaccine according to any one of claims 17 to 19.

37. The method of inducing immunity according to claim 34, wherein the vaccine comprises a complex of a target antigen and an antibody.

38. The method of inducing immunity according to claim 37, wherein the target antigen is a pathogen antigen or a tumor antigen.

39. A method of treating or preventing a disease, comprising the step of administering an effective amount of a vaccine that is incorporated into an antigen-presenting cell via binding to an Fc receptor and an effective amount of the adjuvant according to any one of claims 29 to 32 to a subject in need thereof.

40. A method of inducing immunity, comprising the step of administering an anti-tumor antigen antibody and the adjuvant according to any one of claims 29 to 32 to a subject in need thereof.

41. A method of inducing immunity, comprising the step of administering an anti-tumor cell antibody and the adjuvant according to any one of claims 29 to 32 to a subject in need thereof.

42. A method of inducing immunity, comprising the step of administering an anticancer agent and the adjuvant according to any one of claims 29 to 32 to a subject in need thereof.

43. A method of treating or preventing a disease, comprising the step of administering an effective amount of an anti-tumor antigen antibody and an effective amount of the adjuvant according to any one of claims 29 to 32 to a subject in need thereof.


SEQUENCE LISTING

<110>  Riken

<120>  Novel anti-HSP90 monoclonal antibody

<130>  091698

<150>  US 61/323,578
<151>  2010-04-13

<160>  12

<170>  PatentIn version 3.3

<210>  1
<211>  12
<212>  PRT
<213>  Artificial

<220>
<223>  Epitope presented in Hsp90


<220>
<221>  MISC_FEATURE
<222>  (2)..(2)
<223>  Xaa stands for any amino acid.

<220>
<221>  MISC_FEATURE
<222>  (3)..(3)
<223>  Xaa stands for any amino acid.

<220>
<221>  MISC_FEATURE
<222>  (5)..(5)
<223>  Xaa stands for any amino acid.

<220>
<221>  MISC_FEATURE
<222>  (12)..(12)
<223>  Xaa stands for any amino acid.

<400>  1

Val Xaa Xaa Glu Xaa Pro Pro Leu Glu Gly Asp Xaa
1               5                   10


<210>  2
<211>  12
<212>  PRT
<213>  Artificial

<220>
<223>  Epitope presented in Hsp90


<220>
<221>  MISC_FEATURE
<222>  (2)..(2)
<223>  Xaa stands for any amino acid.

<220>

<221>  MISC_FEATURE
<222>  (4)..(4)
<223>  Xaa stands for any amino acid.

<400>  2

His Xaa Ile Xaa Glu Thr Leu Arg Gln Lys Ala Glu
1               5                   10


<210>  3
<211>  12
<212>  PRT
<213>  Homo sapiens

<400>  3

Val Thr Glu Glu Met Pro Pro Leu Glu Gly Asp Asp
1               5                   10


<210>  4
<211>  12
<212>  PRT
<213>  Homo sapiens

<400>  4

Val Pro Asp Glu Ile Pro Pro Leu Glu Gly Asp Glu
1               5                   10


<210>  5
<211>  12
<212>  PRT
<213>  Homo sapiens

<400>  5

His Ser Ile Ile Glu Thr Leu Arg Gln Lys Ala Glu
1               5                   10


<210>  6
<211>  12
<212>  PRT
<213>  Homo sapiens

<400>  6

His Pro Ile Val Glu Thr Leu Arg Gln Lys Ala Glu
1               5                   10


<210>  7
<211>  12
<212>  DNA
<213>  Artificial

<220>
<223>  Primer for cDNA synthesis

<400>  7
tccakagttc ca

<210>  8
<211>  12
<212>  DNA
<213>  Artificial

<220>
<223>  Primer for cDNA synthesis

<400>  8
gctgtcctga tc                                                          12


<210>  9
<211>  557
<212>  DNA
<213>  Mus musculus


<220>
<221>  CDS
<222>  (1)..(555)

<400>  9
atg atc agt gtc ctc tct aca cag tcc ctg aca aca ctg act cta acc        48
Met Ile Ser Val Leu Ser Thr Gln Ser Leu Thr Thr Leu Thr Leu Thr
1               5                   10                  15

atg gga tgg agc cgg atc ttt ctc ttc ctc ctg tca ata att gca ggt        96
Met Gly Trp Ser Arg Ile Phe Leu Phe Leu Leu Ser Ile Ile Ala Gly
            20                  25                  30

gtc cat tgc cag gtc cag ctg cag cag tct gga cct gag ctg gtg aag       144
Val His Cys Gln Val Gln Leu Gln Gln Ser Gly Pro Glu Leu Val Lys
        35                  40                  45

cct ggg gct tca gtg agg ata tcc tgc aag gct tct ggc tac acc ttc       192
Pro Gly Ala Ser Val Arg Ile Ser Cys Lys Ala Ser Gly Tyr Thr Phe
    50                  55                  60

aca agc tac tat ata cac tgg gtg aag cag agg cct gga cag gga ctt       240
Thr Ser Tyr Tyr Ile His Trp Val Lys Gln Arg Pro Gly Gln Gly Leu
65                  70                  75                  80

gag tgg att gga tgg att tat cct gga aat gtt aat act aag tac aat       288
Glu Trp Ile Gly Trp Ile Tyr Pro Gly Asn Val Asn Thr Lys Tyr Asn
                85                  90                  95

gag aag ttc aag ggc aag gcc aca ctg act gca gac aaa tcc tcc agc       336
Glu Lys Phe Lys Gly Lys Ala Thr Leu Thr Ala Asp Lys Ser Ser Ser
            100                 105                 110

aca gcc tac atg cag ctc agc agc ctg acc tct gag gac tct gcg gtc       384
Thr Ala Tyr Met Gln Leu Ser Ser Leu Thr Ser Glu Asp Ser Ala Val
        115                 120                 125

tat ttc tgt gca aga tac ggt aac tac ccg ttt gct tac tgg ggc caa       432
Tyr Phe Cys Ala Arg Tyr Gly Asn Tyr Pro Phe Ala Tyr Trp Gly Gln
    130                 135                 140

ggg act ctg gtc act gtc tct gca gcc aaa aca aca gcc cca tcg gtc       480
Gly Thr Leu Val Thr Val Ser Ala Ala Lys Thr Thr Ala Pro Ser Val
145                 150                 155                 160

tat cca ctg gcc cct gtg tgt gga gat aca act ggc tcc tcg gtg act       528

Tyr Pro Leu Ala Pro Val Cys Gly Asp Thr Thr Gly Ser Ser Val Thr
                165                 170                 175

cta gga tgc ctg gtc aag ggt tat ttc cc                            557
Leu Gly Cys Leu Val Lys Gly Tyr Phe
            180                 185

<210>  10
<211>  185
<212>  PRT
<213>  Mus musculus

<400>  10

Met Ile Ser Val Leu Ser Thr Gln Ser Leu Thr Thr Leu Thr Leu Thr
1               5                   10                  15

Met Gly Trp Ser Arg Ile Phe Leu Phe Leu Leu Ser Ile Ile Ala Gly
            20                  25                  30

Val His Cys Gln Val Gln Leu Gln Gln Ser Gly Pro Glu Leu Val Lys
        35                  40                  45

Pro Gly Ala Ser Val Arg Ile Ser Cys Lys Ala Ser Gly Tyr Thr Phe
    50                  55                  60

Thr Ser Tyr Tyr Ile His Trp Val Lys Gln Arg Pro Gly Gln Gly Leu
65                  70                  75                  80

Glu Trp Ile Gly Trp Ile Tyr Pro Gly Asn Val Asn Thr Lys Tyr Asn
                85                  90                  95

Glu Lys Phe Lys Gly Lys Ala Thr Leu Thr Ala Asp Lys Ser Ser Ser
            100                 105                 110

Thr Ala Tyr Met Gln Leu Ser Ser Leu Thr Ser Glu Asp Ser Ala Val
        115                 120                 125

Tyr Phe Cys Ala Arg Tyr Gly Asn Tyr Pro Phe Ala Tyr Trp Gly Gln
    130                 135                 140

Gly Thr Leu Val Thr Val Ser Ala Ala Lys Thr Thr Ala Pro Ser Val
145                 150                 155                 160

Tyr Pro Leu Ala Pro Val Cys Gly Asp Thr Thr Gly Ser Ser Val Thr
                165                 170                 175

Leu Gly Cys Leu Val Lys Gly Tyr Phe
            180                 185

<210>  11
<211>  532

<212>  DNA
<213>  Mus musculus

<220>
<221>  CDS
<222>  (1)..(531)

<400>  11
atg aag ttg cct gtt agg ctg ttg gtg ctg atg ttc tgg att cct gct      48
Met Lys Leu Pro Val Arg Leu Leu Val Leu Met Phe Trp Ile Pro Ala
1               5                   10                  15

tcc agc agt gat gtt gtg atg acc caa act cca ctc tcc ctg cct gtc      96
Ser Ser Ser Asp Val Val Met Thr Gln Thr Pro Leu Ser Leu Pro Val
            20                  25                  30

agt ctt gga gat caa gcc tcc atc tct tgc aga tct agt cag agc ctt      144
Ser Leu Gly Asp Gln Ala Ser Ile Ser Cys Arg Ser Ser Gln Ser Leu
        35                  40                  45

gta cac agt aat gga aac acc tat tta cat tgg tac ctg cag aag cca      192
Val His Ser Asn Gly Asn Thr Tyr Leu His Trp Tyr Leu Gln Lys Pro
    50                  55                  60

ggc cag tct cca aag ctc ctg atc tac aaa gtt tcc aac cga ttt tct      240
Gly Gln Ser Pro Lys Leu Leu Ile Tyr Lys Val Ser Asn Arg Phe Ser
65                  70                  75                  80

ggg gtc cca gac agg ttc agt ggc agt gga tca ggg aca gat ttc aca      288
Gly Val Pro Asp Arg Phe Ser Gly Ser Gly Ser Gly Thr Asp Phe Thr
                85                  90                  95

ctc aag atc agc aga gtg gag gct gag gat ctg gga gtt tat ttc tgc      336
Leu Lys Ile Ser Arg Val Glu Ala Glu Asp Leu Gly Val Tyr Phe Cys
            100                 105                 110

tct caa agt aca cat gtt cct ccg acg ttc ggt gga ggc acc aag ctg      384
Ser Gln Ser Thr His Val Pro Pro Thr Phe Gly Gly Gly Thr Lys Leu
        115                 120                 125

gaa atc aaa cgg gct gat gct gca cca act gta tcc atc ttc cca cca      432
Glu Ile Lys Arg Ala Asp Ala Ala Pro Thr Val Ser Ile Phe Pro Pro
    130                 135                 140

tcc agt gag cag tta aca tct gga ggt gcc tca gtc gtg tgc ttc ttg      480
Ser Ser Glu Gln Leu Thr Ser Gly Gly Ala Ser Val Val Cys Phe Leu
145                 150                 155                 160

aac aac ttc tac ccc aaa gac atc aat gtc aag tgg aag att gat ggc      528
Asn Asn Phe Tyr Pro Lys Asp Ile Asn Val Lys Trp Lys Ile Asp Gly
                165                 170                 175

agt g                                                                532
Ser

<210>  12
<211>  177
<212>  PRT
<213>  Mus musculus

<400>  12

Met Lys Leu Pro Val Arg Leu Leu Val Leu Met Phe Trp Ile Pro Ala
1               5                   10                  15

Ser Ser Ser Asp Val Val Met Thr Gln Thr Pro Leu Ser Leu Pro Val
            20                  25                  30

Ser Leu Gly Asp Gln Ala Ser Ile Ser Cys Arg Ser Ser Gln Ser Leu
        35                  40                  45

Val His Ser Asn Gly Asn Thr Tyr Leu His Trp Tyr Leu Gln Lys Pro
    50                  55                  60

Gly Gln Ser Pro Lys Leu Leu Ile Tyr Lys Val Ser Asn Arg Phe Ser
65                  70                  75                  80

Gly Val Pro Asp Arg Phe Ser Gly Ser Gly Ser Gly Thr Asp Phe Thr
                85                  90                  95

Leu Lys Ile Ser Arg Val Glu Ala Glu Asp Leu Gly Val Tyr Phe Cys
            100                 105                 110

Ser Gln Ser Thr His Val Pro Pro Thr Phe Gly Gly Gly Thr Lys Leu
        115                 120                 125

Glu Ile Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser